# EUROPEAN PATENT SPECIFICATION

(11) **EP 3 975 955 B1**
(45) Date of publication and mention of the grant of the patent: **01.11.2023**
(21) Application number: 20727859.9
(22) Date of filing: 21.05.2020
(51) Int. Cl.: A61F 13/02, A61F 13/00

(54) **MEDICAL DRESSING FOR SECURING A TUBIFORM COMPONENT OF A MEDICAL DEVICE**
MEDIZINISCHER VERBAND ZUR BEFESTIGUNG EINER TUBENFÖRMIGEN KOMPONENTE EINES MEDIZINPRODUKTS
PANSEMENT MÉDICAL PERMETTANT DE FIXER UN COMPOSANT TUBIFORME D'UN DISPOSITIF MÉDICAL

(30) Priority: 28.05.2019 US 201962853192 P
(43) Date of publication of application: 06.04.2022
(73) Proprietor: 3M Innovative Properties Co., St. Paul, MN 55133-3427 (US)
(72) Inventor: SIERACKI, James M., Saint Paul, Minnesota 55133-3427 (US); SCHEIBEL, Krystal J., Saint Paul, Minnesota 55133-3427 (US)
(74) Representative: Vossius & Partner Patentanwälte Rechtsanwälte mbB
(86) International application number: PCT/IB2020/054836
(87) International publication number: WO 2020/240362

(56) References cited:
- EP-A1- 2 902 068
- EP-A1- 3 015 129
- WO-A1-2014/194786
- WO-A1-2018/089563

## Description

### Technical Field

The present disclosure relates to a conformable medical dressing for securing a tubiform component of a medical device to a receiving surface.

### Background

Venous, arterial, and body fluid catheters are commonly used by physicians. For example, such catheters may be used to gain access to the vascular system for dialysis, for introducing pharmaceutical agents, for nutrition or fluids, for hemodynamic monitoring, and for blood draws. Alternatively, catheters can be used for drainage of fluid collections and to treat infection. Alternatively, catheters can contain electrical leads for neuro-stimulation, cardiac pacing, and the like. Following introduction into the patient, the catheter is secured to the patient, generally at a point near the entry into the patient's body.

In many instances, the catheter is commonly secured to the patient using an adhesive tape on the skin or by suturing a catheter hub to the patient's skin. In other circumstances, the catheter may be secured to the patient using a subcutaneous anchor mechanism (such as an anchor sleeve equipped with anchors that are deployed using an external actuator handle or a separate delivery device). In many cases, the medical practitioner will make efforts to clean the skin area around the catheter insertion site for purposes of a patient's comfort, safety, and improved visualization of the catheter insertion site after the catheter is installed.
EP 2 902 068 A1 relates to an apparatus for cleansing wounds in which irrigant fluid from a reservoir connected to a conformable wound dressing and wound exudate from the dressing are recirculated by a device for moving fluid through a flow path which passes through the dressing and a means for fluid cleansing and back to the dressing. The cleansing means removes materials deleterious to wound healing, and the cleansed fluid, still containing materials that are beneficial in promoting wound healing, is returned to the wound bed.
WO 2018/089563 A1 relates to trimmable conformable wound dressings, kits containing the same and methods of using the same. The wound dressings may protect a treatment site from microbial contamination or other disturbances while a wound heals or to enable the application and maintenance of vacuum over the treatment site. The wound dressings may include first and second sections of support layer material on opposite sides of one or more intermediate sections of support layer material. The first and second sections of support layer material meet the intermediate sections of support layer material along peel tab junctions that provide peel tabs.
WO 2014/194786 A1 relates to a multi-layer foam dressing for negative pressure sealing drainage, comprising a negative pressure conduction layer and a drainage protection layer provided at the lower part of the negative pressure conduction layer and used for contacting with a wound surface or a wound cavity. The negative pressure conduction layer and the drainage protection layer are both porous foam material layers. The negative pressure conduction layer is a hydrophobic porous foam material layer, the drainage protection layer is a hydrophilic porous foam material layer, and the two layers of materials are fixedly connected and have the pores thereof interpenetrated and in communication with each other.
EP 3 015 129 A1 relates to an attachment device for securing an elongated element on the body of a patient. The device includes a retaining support for retaining the elongated element, including at least one lower wall and an adhesive strip bearing the retaining support, and intended to be applied on the skin of the patient, the adhesive strip defining at least one region of the attachment device shifted with respect to the lower wall. A region of the attachment device located under the lower wall along an axis for inserting the elongated element into the retaining support is intended to be non-adherent to the skin of the patient. The retaining support comprises a clasping flap able to be maneuvered with respect to the lower wall between an open position and a clasping position of the elongated element. The elongated element is intended to be maintained between the lower wall and the clasping flap.

### Summary

The invention for which protection is sought is defined by the independent claim. The dependent claims concern particular embodiments.

Medical devices comprising a tubiform component (e.g., a catheter) are at risk of being dislodged from the patient if the tubiform component becomes entangled with a person or object, and thereby the tubiform component is subjected to a force moving in a direction away from the patient. A variety of devices have been used to secure tubiform components to a patient, each device having its own limitations. Tapes and/or elastic dressings are commonly used because of their comfort and conformability to anatomic features of the patient's body.

However, the instant inventors have discovered that certain forces applied to tapes and medical dressings securing a tubiform component can result in loss of adhesion to skin proximate the area(s) where the the edge(s) of the dressing contact the tubiform component. This loss of adhesion to skin (or other surfaces) at the edge of the tape or dressing can propagate and ultimately result in the loss of adhesion to the patient (or other surfaces) of most or all of the tape or dressing.

The disclosed medical tapes and dressings substantially reduce the likelihood of the loss of adhesion proximate the edge of a tape or dressing that contacts a tubiform component (e.g., a wire and/or catheter) that extends under a portion of the tape or dressing. In addition, visual cues provided by components of the inventive medical tapes and dressings of the present disclosure improve the likelihood of securing the tubiform component in the proper orientation relative to the tape or dressing and improve the likelihood of correctly applying the tape or dressing to the patient.

Disclosed is a medical dressing that comprises a body and an adhesive. The body can comprise a first major surface; a second major surface opposite the first major surface; a perimeter; a central region; a first flap defined by a distal end formed by a first part of the perimeter, a first indent having a first end proximate the central region, a second indent having a second end proximate the central region, and a first hinge region extending between the first end and the second end; a first lateral extension extending in a first direction away from the first flap; and a second lateral extension extending in a second direction away from the first flap, wherein the first direction is substantially opposite the second direction. The first flap has a maximum length and a maximum width. A ratio of the maximum width to the maximum length is less than 4 to 1. The adhesive can be disposed on at least a portion of the second major surface. The portion of the second major surface on which the adhesive is disposed can include a part of each of the first flap, the first lateral extension, and the second lateral extension.

In another aspect of the medical dressing the body can comprise a first major surface; a second major surface opposite the first major surface; a perimeter; a central region; a first flap defined by a distal end formed by a first part of the perimeter, a first indent having a first end proximate the central region, a second indent having a second end proximate the central region, and a first hinge region extending between the first end and the second end; a first lateral extension extending in a first direction away from the first flap; and a second lateral extension extending in a second direction away from the first flap, wherein the first direction is substantially opposite the second direction. The adhesive can be disposed on at least a portion of the second major surface. The portion of the second major surface on which the adhesive is disposed can include a part of each of the first flap, the first lateral extension, and the second lateral extension. The first flap defines a first flap axis extending from the perimeter through the first hinge region. Proximate the first end, the first indent can comprise a portion that slants in a direction away from the first flap axis or wherein, proximate the second end, the second indent comprises a portion that slants in a direction away from the first flap axis; or proximate the first end, the first indent comprises a portion that slants in a direction toward the first flap axis or wherein, proximate the second end, the second indent comprises a portion that slants in a direction toward from the first flap axis.

In any of the above embodiments, the body of the medical dressing further can comprise a second flap defined by a distal end formed by a second part of the perimeter, a third indent having a third end proximate the central region, a fourth indent having a fourth end proximate the central region, and a second hinge region extending between the third end and the fourth end. a third lateral extension extending in a third direction away from the second flap; and a fourth lateral extension extending in a fourth direction away from the second flap, wherein the third direction is substantially opposite the fourth direction. The first part of the perimeter is located on the perimeter opposite the second part of the perimeter. The portion of the second major surface on which the adhesive is disposed includes a part of the second flap, the third lateral extension, and the fourth lateral extension.

In yet another aspect, the present disclosure provides a tape. The tape can comprise a plurality of the medical dressings of any one of the above embodiments, wherein the adhesive of each medical dressing of the plurality is releasably adhered to a unitary liner.

In yet another aspect, the present disclosure provides a medical tape. The medical tape comprises a body and an adhesive. The body can comprise a first major surface, a second major surface opposite the first major surface, a first primary edge, a second primary edge, a longitudinal axis, and a plurality of body units. Each body unit of the plurality of body units can comprise a first flap defined by a distal end formed by a part of the first primary edge or a part of the secondary edge, a first indent having a first end proximate the longitudinal axis, a second indent having a second end proximate the longitudinal axis, and a first hinge region extending between the first end and the second end; a first lateral extension extending in a first direction away from the first flap; and a second lateral extension extending in a second direction away from the first flap, wherein the first direction is substantially opposite the second direction. Each of the body units is joined to at least one adjacent body unit at an area of weakness that extends substantially orthogonal to the longitudinal axis. The adhesive is disposed on at least a portion of the second major surface, wherein the portion of the second major surface on which the adhesive is disposed includes a part of each of the first flap, the first lateral extension, and the second lateral extension of each of the body units.

In any of the above embodiments of the medical tape, the distal end of the first flap is formed by a part of the first primary edge. In these embodiments, each body unit further can comprise a second flap defined by a distal end formed by a second part of the second primary edge, a third indent having a third end proximate the longitudinal axis, a fourth indent having a fourth end proximate the longitudinal axis, and a second hinge region extending between the third end and the fourth end; a third lateral extension extending in a third direction away from the second flap; and a fourth lateral extension extending in a fourth direction away from the second flap, wherein the third direction is substantially opposite the fourth direction. The first part of the first primary edge is located on the body opposite the second part of the second primary edge. The portion of the second major surface on which the adhesive is disposed includes a part of each second flap of each of the body units.

In yet another aspect, the present disclosure provides a system. The system can comprise any of the above embodiments of a medical dressing and a medical device comprising a tubiform component, wherein the adhesive disposed on the second major surface of the first flap of the medical dressing contacts the tubiform component.

In yet another aspect, the present disclosure provides a system. The system can comprise any of the above embodiments of a body unit of a medical tape and a medical device comprising a tubiform component, wherein the adhesive disposed on the second major surface of the first flap of the body unit contacts the tubiform component.

In yet another aspect, the present disclosure provides a method of securing a medical device comprising a tubiform component to a receiving surface. The method can comprise contacting an adhesive disposed on a second major surface of a first flap of a medical dressing with a first part of an outer surface of the tubiform component of the medical device. The medical device can comprise a body and an adhesive. The body can comprise a first major surface; a second major surface opposite the first major surface; a perimeter; a central region; a first flap defined by a distal end formed by a first part of the perimeter, a first indent having a first end proximate the central region, a second indent having a second end proximate the central region, and a first hinge region extending between the first end and the second end; a first lateral extension extending in a first direction away from the first flap; and a second lateral extension extending in a second direction away from the first flap, wherein the first direction is substantially opposite the second direction. The adhesive can be disposed on at least a portion of the second major surface, wherein the portion of the second major surface on which the adhesive is disposed includes a part of each of the first flap, the first lateral extension, and the second lateral extension. The method further can comprise contacting the adhesive disposed on the second major surface of the first lateral extension of the medical dressing with a first part of the receiving surface, and contacting the adhesive disposed on the second major surface of the second lateral extension of the medical dressing with a second part of the receiving surface.

In yet another aspect, the present disclosure provides a method of securing a medical device comprising a tubiform component to a receiving surface. The method can comprise contacting an adhesive disposed on a second major surface of a first flap of a body unit of a medical tape with a first part of an outer surface of the tubiform component of the medical device. The body unit can comprise a first flap defined by a distal end formed by a part of the first primary edge or a part of the secondary edge, a first indent having a first end proximate the longitudinal axis, a second indent having a second end proximate the longitudinal axis, and a first hinge region extending between the first end and the second end; a first lateral extension extending in a first direction away from the first flap; a second lateral extension extending in a second direction away from the first flap, wherein the first direction is substantially opposite the second direction; and an adhesive disposed on at least a portion of the second major surface, wherein the portion of the second major surface on which the adhesive is disposed includes a part of each of the first flap, the first lateral extension, and the second lateral extension. The medical tape comprises a plurality of body units, each body unit detachably attached to an adjacent body unit. The method further can comprise contacting the adhesive disposed on the second major surface of the first lateral extension of the medical dressing with a first part of the receiving surface, and contacting the adhesive disposed on the second major surface of the second lateral extension of the medical dressing with a second part of the receiving surface.

"Elastic" means a material able to elongate and regain some or all of its original shape.

"Slit" means a long, narrow through cut. In one embodiment, the slit has a length that is significantly longer than a width. In one embodiment, the slit has a length at least 5 times greater than a width. In one embodiment, the slit has a length at least 10 times greater than a width. In one embodiment, the slit has essentially no width.

The words "preferred" and "preferably" refer to embodiments that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments.

The terms "comprises", and variations thereof, do not have a limiting meaning where these terms appear in the description and claims.

As used herein, "a," "an," "the," "at least one," and "one or more" are used interchangeably. The term "and/or" means one or all of the listed elements (e.g., preventing and/or treating an affliction means preventing, treating, or both treating and preventing further afflictions).

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### Brief Description of Drawings

The invention will be further described with reference to the drawings, wherein corresponding reference characters indicate corresponding parts throughout the several views, and wherein:
FIG. 1A is a schematic plan view of one embodiment of a medical dressing comprising a first flap according to the present disclosure.
FIG. 1B is a schematic side view of the medical dressing of FIG. 1A.
FIG. 2A is a schematic plan view of an alternative embodiment of a medical dressing comprising a first flap according to the present disclosure.
FIG. 2B is a schematic side view of the medical dressing of FIG. 2A.
FIG. 3A is a schematic plan view of a medical dressing comprising two layers and a first flap according to the present disclosure.
FIG 3B is a schematic side view of the medical dressing of FIG. 3A.
FIG. 4A is a schematic plan view of a medical dressing comprising two layers, a first flap, and a viewing window according to the present disclosure.
FIG. 4B is a schematic side view of the medical dressing of FIG. 4A.
FIG, 4C is a schematic cross-sectional side view, along line 4C-4C, of the medical dressing of FIG. 4A.
FIG. 5 is a schematic plan view of one embodiment of a medical dressing comprising a first flap and a second flap according to the present disclosure.
FIG. 6A is a schematic plan view of one embodiment of a tape comprising a plurality of medical dressings according to the present disclosure.
FIG. 6B is a schematic side view of the tape of FIG. 6A.
FIG. 7A is a schematic plan view of one embodiment of a medical tape comprising a plurality of body units, each body unit having a first flap according to the present disclosure.
FIG. 7B is a schematic side view of the tape of FIG. 7A.
FIG. 8A is a schematic plan view of one embodiment of a medical tape comprising a plurality of body units, each body unit having a first flap and a second flap according to the present disclosure.
FIG. 8B is a schematic side view of the tape of FIG. 8A.
FIG. 9A is a schematic plan view of an alternative embodiment of a medical dressing comprising a first flap and a second flap according to the present disclosure.
FIG. 9B is a schematic plan view of the medical dressing of FIG. 9A securing a tubiform component of a medical device to a surface.
FIG. 9C is a schematic side view of the medical dressing and the tubiform component of FIG. 9B.
FIG. 10A is a perspective schematic view of a medical dressing securing a tubiform component of a medical device to a surface according to the present disclosure.
FIG. 10B is a perspective schematic view of the medical dressing and the tubiform component of FIG. 10A wherein a force is applied to the tubiform component in a first direction.
FIG. 10C is a perspective schematic view of the medical dressing and the tubiform component of FIG. 10A wherein a force is applied to the tubiform component in a second direction.
FIG. 11A-11F are schematic plan views of six different embodiments of medical dressings comprising first and second flaps formed in part by indents according to the present disclosure, wherein each flap comprises a flap axis, wherein each of the indents comprises a portion that slants in a direction away from the flap axis or comprises a portion that slants in a direction toward the flap axis.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention.

### Detailed Description

The present disclosure provides medical tapes and medical dressings that may be used to secure a medical device comprising a tubiform component to a surface. Methods of use of the medical tapes and medical dressings are also provided. Advantageously, the medical tapes and medical dressings can provide improved adherence of the tubiform component to the surface even when the tubiform component is subjected to a force that would otherwise draw it away from the surface.

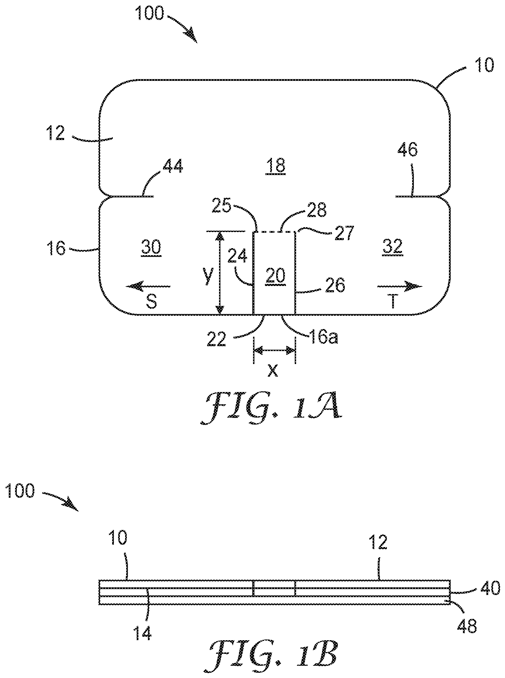

In one aspect, the present disclosure provides a medical dressing. Turning now to the drawings, FIG. 1A-B are various views of one embodiment of a medical dressing 100 according to the present disclosure. The medical dressing 100 comprises a body 10 that has a first major surface 12, a second major surface 14 opposite the first major surface 12, a perimeter 16, and a central region 18.

The body 10 of the medical dressing 100 further comprises a first flap 20. The first flap 20 is defined by a first distal end 22, a first indent 24 having a first end 25 proximate the central region 18, a second indent 26 having a second end 27 proximate the central region 18, and a first hinge region 28 extending between the first end 25 and the second end 27. The first distal end 22 is formed by a first part 16a of the perimeter 16 of the body 10. In any embodiment of a medical dressing disclosed herein, a portion of the first indent 24 or the entire first indent 24 may comprise a slit (i.e., a thin cut extending through the body 10). In any embodiment of any medical dressing disclosed herein, a portion of the second indent 26 or the entire second indent 26 may comprise a slit (i.e., a thin cut extending through the body 10).

In any embodiment (not shown) of a medical dressing disclosed herein, a portion of the first indent or the entire first indent may comprise a plurality of perforations extending from the perimeter to the first end proximate the central region. The perforations may comprise small holes and/or small slits generally aligned along a path to form the first indent. In any embodiment of any medical dressing disclosed herein, a portion of the second indent or the entire second indent may comprise a plurality of perforations extending from the perimeter to the second end proximate the central region. The perforations may comprise small holes and/or small slits generally aligned along a path to form the second indent. Advantageously, the perforations can be torn by the operator before, during, or after applying the dressing to the tubiform component or to the surface. Moreover, the perforated indent advantageously provides a visual cue that can be used by the operator to align the dressing with the tubiform component before and/or during the process of using the dressing to secure the tubiform component.

The first flap has a maximum width "x" and a maximum length "y". In certain embodiments the ratio ("r") of the maximum width of the first flap 20 to the maximum length of the first flap 20 is less than 4 to 1. In some embodiments, the ratio "r" is less than 3.5 to 1. In some embodiments, the ratio "r" is less than 3 to 1. In some embodiments, the ratio "r" is less than 2.5 to 1. In some embodiments, the ratio "r" is less than 2 to 1. In some embodiments, the ratio "r" is less than 1.5 to 1. In some embodiments, the ratio "r" is less than 1 to 1. In some embodiments, the ratio "r" is less than 0.75 to 1. In some embodiments, the ratio "r" is less than 0.5 to 1.

In any embodiment of a medical dressing according to the present disclosure, and as discussed in more detail below, the first flap defines a first flap axis extending from the perimeter to the hinge region. In these embodiments, proximate the first end of the first indent, the first indent optionally comprises a portion that slants in a direction away from the first flap axis.

In any embodiment of a medical dressing according to the present disclosure, and as discussed in more detail below, the second flap defines a second flap axis extending from the perimeter to the hinge region. In these embodiments, proximate the second end of the second indent, the second indent optionally comprises a portion that slants in a direction away from the second flap axis.

The body 10 of the medical dressing 100 further comprises a first lateral extension 30 extending in a first direction ("S") away from the first flap 20 and a second lateral extension 32 extending in a second direction ("T") away from the first flap. The first direction "S" is substantially opposite the second direction "T". That is, the first direction is oriented greater than 90 degrees to about 180 degrees relative to the second direction. In certain embodiments, the first direction is oriented greater than 120 degrees to about 180 degrees relative to the second direction. In certain embodiments, the first direction is oriented greater than 150 degrees to about 180 degrees relative to the second direction. In certain embodiments, the first direction is oriented about 180 degrees relative to the second direction.

The body 10 of the medical dressing 100 can comprise a sheet material. Suitable sheet materials are well known in the art of medical dressings and include, but are not limited to, a transparent polymeric film, a translucent polymeric film, an opaque polymeric material, a woven fabric, a nonwoven fabric, a foam, or a combination of any two or more of the foregoing sheet materials. The material from which the body is constructed generally is flexible enough to conform to the shape of a medical device and to a surface (e.g., a generally flat surface, an uneven surface) to which the medical device is to be attached via the medical dressing. Other aspects of the body 10 of the medical device 100 are discussed elsewhere in the specification.

In some embodiments, the material from which the body 10 is constructed is flexible, but also has enough rigidity to be self-supporting. Advantageously, such materials (e.g., a relatively thick polymeric film; a laminate of a polymeric film and a woven material, a non-woven material, and/or a foam) are rigid enough to prevent the dressing from unintentionally folding back on itself when applying the dressing to a medical device. In any embodiment, a material from which the body is constructed may be an elastic material.

The medical dressing 100 further comprises an adhesive 40 disposed on at least a portion of the second major surface 14. The portion of the second major surface on which the adhesive is disposed includes at least a part of each of the first flap, the first lateral extension, and the second lateral extension. In the illustrated embodiment of FIGS. 1A and 1B, the adhesive 40 is shown as a layer (e.g., a uniform layer) adhered to the second major surface 14 of the body 10 of the medical dressing 100. Other aspects of the adhesive 40 of the medical device 100 are discussed elsewhere in the specification.

Optionally, in any embodiment, the medical dressing 100 can comprise at least one indent (e.g., fifth indent 44) extending from the perimeter 16 into or adjacent the first lateral extension 30. Advantageously, the fifth indent 44 provides greater conformability of the medical dressing 100 to an uneven surface and may reduce the likelihood of separation of the edge of the dressing from a skin surface when the underlying tissue (e.g., muscle tissue) is subjected to expansion or contraction. In any embodiment, a portion of the fifth indent 44 or the entire fifth indent 44 may comprise a slit (i.e., a thin cut extending through the body 10). In any embodiment (not shown) of a medical dressing disclosed herein, a portion of the fifth indent or the entire fifth indent may comprise a plurality of perforations extending from the perimeter into the first lateral extension. The perforations may comprise small holes and/or small slits generally aligned along a path to form the fifth indent. Advantageously, the perforations can be torn by the operator before or during the application of the dressing to the tubiform component or to the surface.

In addition, in any embodiment, the medical dressing 100 optionally can comprise at least one indent (e.g., sixth indent 46) extending from the perimeter 16 into or adjacent the second lateral extension 32. Advantageously, the sixth indent 46 provides greater conformability of the medical dressing 100 to an uneven surface and may reduce the likelihood of separation of the edge of the dressing from a skin surface when the underlying tissue (e.g., muscle tissue) is subjected to expansion or contraction. In any embodiment, a portion of the sixth indent 46 or the entire sixth indent 46 may comprise a slit (i.e., a thin cut extending through the body 10). In any embodiment of any medical dressing disclosed herein, a portion of the sixth indent or the entire sixth indent may comprise a plurality of perforations extending from the perimeter into the second lateral extension. The perforations may comprise small holes and/or small slits generally aligned along a path to form the sixth indent. Advantageously, the perforations can be torn by the operator before or during the application of the dressing to the tubiform component or to the surface.

In any embodiment of a medical dressing according to the present disclosure, the medical dressing (e.g., medical dressing 100) optionally can comprise a liner 48 as shown in FIG. 1B. The liner 48 covers all (as shown in FIG. 1B) or a portion of the adhesive 40 to prevent contamination of the adhesive. Other aspects of the adhesive 40 of the medical device 100 are discussed elsewhere in the specification. It is contemplated that the liner 48 may comprise optional indents, perforations, or slits (not shown) that optionally may be superimposed by first indent 24 and/or second indent 26 of the body 10.

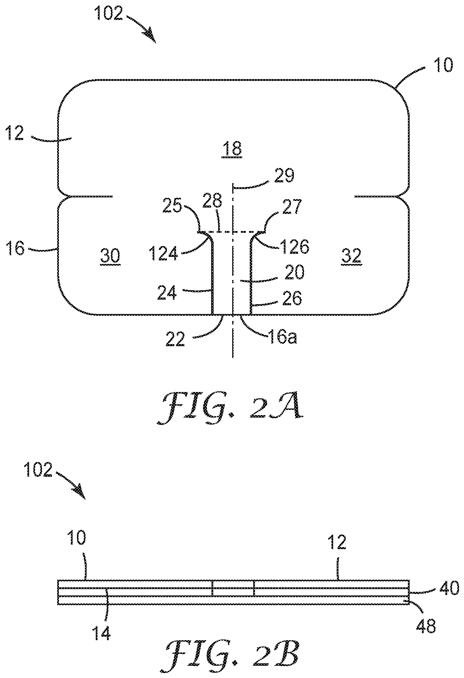

FIG. 2A shows a plan view of an alternative embodiment of a medical dressing 102 according to the present disclosure. The medical dressing 102 comprises a body 10 that has a first major surface 12, a second major surface 14 opposite the first major surface 12, a perimeter 16, and a central region 18. The body 10 of the medical dressing 102 further comprises a first flap 20, a first lateral extension 30 extending in a first direction away from the first flap, and a second lateral extension 32 extending in a second direction away from the first flap, wherein the first direction is substantially opposite the second direction as described above regarding the medical dressing 100. The first flap 20 is defined by a first distal end 22, a first indent 24 having a first end 25 proximate the central region 18, a second indent 26 having a second end 27 proximate the central region 18, and a first hinge region 28 extending between the first end 25 and the second end 27. The first distal end 22 is formed by a first part 16a of the perimeter 16 of the body 10.

The first flap 20 of the medical dressing 102 defines a first flap axis 29 extending from the perimeter 16 through the first hinge region 28. In contrast to the first indent 24 of the medical dressing 100, proximate the first end 25, the first indent 24 of the body 10 of the medical dressing 102 comprises a portion 124 that slants in a direction away from the first flap axis 29 (as shown in FIG. 2) or that slants in a direction toward the first flap axis 29. Optionally, proximate the second end 27, the second indent 26 of the body 10 of the medical dressing 102 comprises a portion 126 that slants in a direction away from the first flap axis 29 (as shown in FIG. 2) or that slants in a direction toward the first flap axis 29.

In addition, the medical dressing 102 further comprises an adhesive 40 disposed on at least a portion of the second major surface of the body. The portion of the second major surface on which the adhesive is disposed includes at least a part of each of the first flap, the first lateral extension, and the second lateral extension. The adhesive has similar properties and can be constructed of materials like those described hereinabove for the medical dressing 100.

Also shown in FIG. 2B is an optional liner 48 as described above for the medical dressing 100. The liner 48 covers all (as shown in FIG. 2B) or a portion of the adhesive 40 to prevent contamination of the adhesive. Other aspects of the adhesive 40 of the medical device 102 are discussed elsewhere in the specification. It is contemplated that the liner 48 may comprise optional indents, perforations or slits (not shown) that optionally may be superimposed by first indent 24 and/or second indent 26 of the body 10.

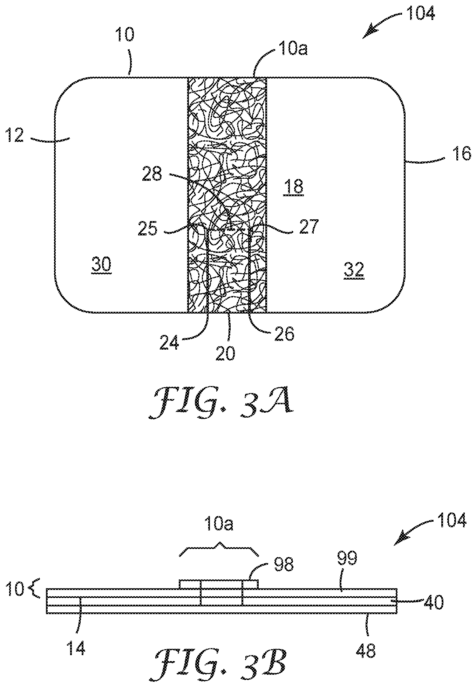

In any embodiment of a medical dressing according to the present disclosure, at least a portion of the body of the medical dressing can comprise a first layer overlaying a second layer. FIG3A-B show various views of one embodiment of a medical dressing 104 comprising a body 10 that comprises a first layer 98 overlaying a second layer 99. The first layer 98 is bonded (e.g., heat-bonded and/or adhesively bonded) to the second layer 99. The first layer 98 can comprise a sheet material such as, for example, a transparent polymeric film, a translucent polymeric film, an opaque polymeric material, a woven fabric, a nonwoven fabric, or a foam. The second layer 99 can comprise a sheet material such as, for example, a transparent polymeric film, a translucent polymeric film, an opaque polymeric material, a woven fabric, a nonwoven fabric, or a foam. Thus, in some embodiments, the first layer 98 and second layer 99 may comprise similar or identical materials. In some embodiments, the first layer 98 and second layer 99 may comprise different materials. In the illustrated embodiment of FIGS 3A-B, the first layer 98 comprises a nonwoven fabric and the second layer 99 comprises a transparent polymeric film.

The medical dressing 104 comprises a body 10 that has a first major surface 12, a second major surface 14 opposite the first major surface 12, a perimeter 16, and a central region 18. The body 10 of the medical dressing 104 further comprises a first flap 20, a first lateral extension 30 extending in a first direction away from the first flap, and a second lateral extension 32 extending in a second direction away from the first flap, wherein the first direction is substantially opposite the second direction as described above regarding the medical dressing 100. The first flap 20 is defined in part by a first indent 24 having a first end 25 proximate the central region 18, a second indent 26 having a second end 27 proximate the central region 18, and a first hinge region 28 extending between the first end 25 and the second end 27. The medical dressing 104 further comprises an adhesive 40 disposed on at least a portion of the second major surface 14. The portion of the second major surface 14 on which the adhesive is disposed includes at least a part of each of the first flap, the first lateral extension, and the second lateral extension, as discussed hereinabove regarding the medical device 100.

In the illustrated embodiment of FIGS. 3A-B, the body 10 includes a portion 10a that comprises a first layer 98 overlaying a second layer 99. The portion 10a includes the first indent 24 (which extends through the first and second layers of the body), the second indent 26 (which extends through the first and second layers of the body) and the hinge area 28. The first layer 98 of the body 10 provides support so that when the first flap 20 is adhesively attached to a tubiform component of a medical device (not shown in FIGS. 3A-B) and an axial stress is applied to the tubiform component, it will prevent the formation of or reduce the extent of a tear propagating from the first end 25 or second end 27.

The first layer defines a first area (not shown in FIGS. 3A-B) that includes area overlapping the second layer as well as area that does not overlap the second layer. The second layer defines a second area (not shown in FIGS. 3A-B) that includes area overlapped the second layer as well as area that is not overlapped by the first layer.

In any embodiment of a medical dressing (e.g., medical dressing 104) comprising a body that includes a first layer and a second layer as described herein, the first area can equal at least 50% of the second area, at least 60% of the second area, at least 70% of the second area, at least 80% of the second area, at least 90% of the second area, at least 95% of the second area, or about 100% of the second area. In any embodiment of a medical dressing (e.g., medical dressing 104) comprising a body that includes a first layer and a second layer as described herein, the first area can equal not more than 95% of the second area, not more than 90% of the second area, not more than 80% of the second area, not more than 70% of the second area, or not more than 60% of the second area.

In any embodiment of a medical dressing (e.g., medical dressing 104) comprising a body that includes a first layer and a second layer as described herein, the second area can equal at least 50% of the first area, at least 60% of the first area, at least 70% of the first area, at least 80% of the first area, at least 90% of the first area, at least 95% of the first area, or about 100% of the first area. In any embodiment of a medical dressing (e.g., medical dressing 104) comprising a body that includes a first layer and a second layer as described herein, the second area can equal not more than 95% of the first area, not more than 90% of the first area, not more than 80% of the first area, not more than 70% of the first area, or not more than 60% of the first area.

In addition, the medical dressing 104 further comprises an adhesive 40 disposed on at least a portion of the second major surface of the body. The portion of the second major surface on which the adhesive is disposed includes at least a part of each of the first flap, the first lateral extension, and the second lateral extension. The adhesive has similar properties and can be constructed of materials similar to those described hereinabove for the medical dressing 100.

Also shown in FIG. 3B is an optional liner 48 as described above for the medical dressing 100. The liner 48 covers all (as shown in FIG. 3B) or a portion of the adhesive 40 to prevent contamination of the adhesive. Other aspects of the adhesive 40 of the medical device 104 are discussed elsewhere in the specification. It is contemplated that the liner 48 may comprise optional indents, perforations or slits (not shown) that optionally may be superimposed by first indent 24 and/or second indent 26 of the body 10.

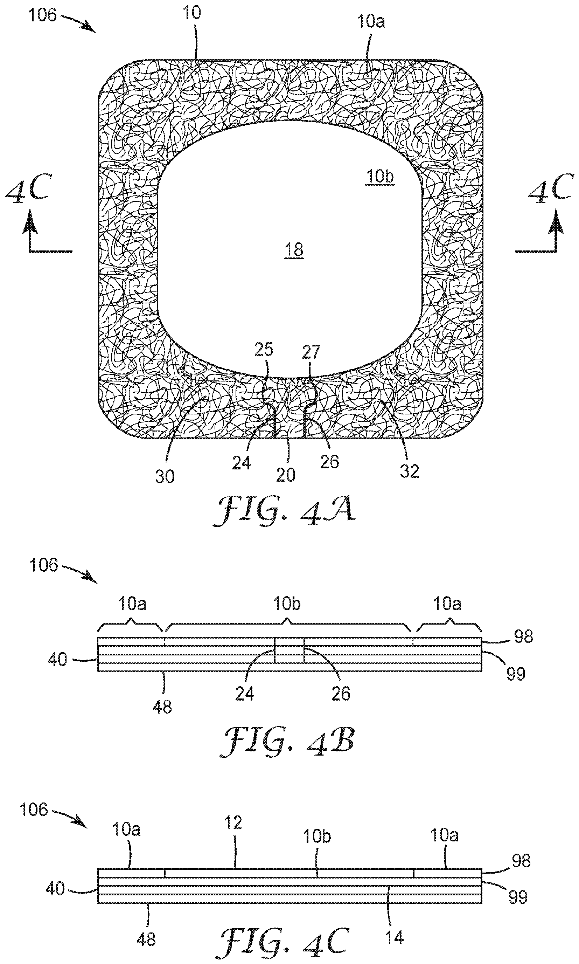

FIGS. 4A-C show various views of an alternative embodiment of a medical dressing 106 comprising a body having a first layer 98 and a second layer 99. The medical dressing 106 comprises a body 10 that has a first major surface 12, a second major surface 14 opposite the first major surface 12, a perimeter 16, and a central region 18. The body 10 of the medical dressing 106 further comprises a first flap 20, a first lateral extension 30 extending in a first direction away from the first flap, and a second lateral extension 32 extending in a second direction away from the first flap, wherein the first direction is substantially opposite the second direction as described above regarding the medical dressing 100. The first flap 20 is defined in part by a first indent 24 having a first end 25 proximate the central region 18, a second indent 26 having a second end 27 proximate the central region 18, and a first hinge region (not shown in FIGS. 4A-C) extending between the first end 25 and the second end 27. The medical dressing 106 further comprises an adhesive 40 disposed on at least a portion of the second major surface 14. The portion of the second major surface 14 on which the adhesive is disposed includes at least a part of each of the first flap, the first lateral extension, and the second lateral extension, as discussed hereinabove with regard to the medical device 100.

In the illustrated embodiment of FIGS. 4A-C, the body 10 includes a portion 10a that comprises a first layer 98 overlaying a second layer 99. The portion 10a includes the first indent 24 (which extends through the first and second layers of the body), the second indent 26 (which extends through the first and second layers of the body) and the hinge area 28. The second layer 99 of the body 10 provides support so that when the first flap 20 is adhesively attached to a tubiform component of a medical device (not shown in FIGS. 4A-C) and an axial stress is applied to the tubiform component, it will prevent the formation of or reduce the extent of a tear propagating from the first end 25 or second end 27.

In the illustrated embodiment of FIGS 4A-C, the first layer 98 comprises a transparent polymeric film fabric and the second layer 99 comprises a nonwoven fabric. In these embodiments, the multilayer portion 10a of the body 10 forms a frame structure that borders the transparent first layer 98, thereby providing a window in the dressing that permits observation of at least a portion of the central region 18. These embodiments can be particularly useful when the tubiform component of the medical device (not shown) is an intravenous catheter, for example, that enters the patient through the skin. Accordingly, the window permits observation of the point of entry of the catheter into the patient (not shown in FIGS. 4A-C).

In addition, the medical dressing 106 further comprises an adhesive 40 disposed on at least a portion of the second major surface of the body. The portion of the second major surface on which the adhesive is disposed includes at least a part of each of the first flap, the first lateral extension, and the second lateral extension. The adhesive has similar properties and can be constructed of materials similar to those described hereinabove for the medical dressing 100.

Also shown in FIGS. 4B-C is an optional liner 48 as described above for the medical dressing 100. The liner 48 covers all or a portion of the adhesive 40 to prevent contamination of the adhesive. Other aspects of the adhesive 40 of the medical device 106 are discussed elsewhere in the specification. It is contemplated that the liner 48 may comprise optional indents, perforations or slits (not shown) that optionally may be superimposed by first indent 24 and/or second indent 26 of the body 10.

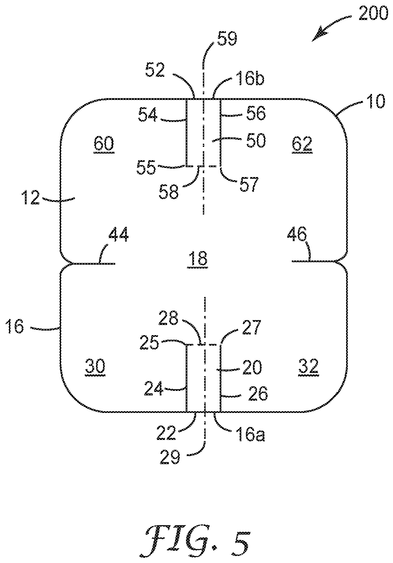

In any embodiment of a medical dressing of the present disclosure, the body of the medical dressing can comprise a second flap. FIG. 5 shows a plan view of one embodiment of a medical dressing 200 comprising a body 10 having a second flap 50.

As described herein regarding the medical dressing 100 shown in FIGS. 1A-B, the medical dressing 200 of the illustrated embodiment of FIG. 5 comprises a body 10 that has a first major surface 12, a second major surface (not shown in FIG. 5) opposite the first major surface 12, a perimeter 16, and a central region 18. In addition, the body 10 of the medical dressing 200 further comprises a first flap 20. The first flap 20 is defined by a first distal end 22, a first indent 24 having a first end 25 proximate the central region 18, a second indent 26 having a second end 27 proximate the central region 18, and a first hinge region 28 extending between the first end 25 and the second end 27. The first distal end 22 is formed by a first part 16a of the perimeter 16 of the body 10. In any embodiment of the medical dressing 200, a portion of the first indent 24 or the entire first indent 24 and/or a portion of the second indent 26 or the entire second indent 26 may comprise a slit (i.e., a thin cut extending through the body 10). The body 10 of the medical dressing 200 further comprises a first lateral extension 30 extending in a first direction away from the first flap 20 and a second lateral extension 32 extending in a second direction away from the first flap 20, as described above for the medical dressing 100. The first direction is substantially opposite the second direction.

In any embodiment (not shown) of a medical dressing disclosed herein, a portion of the first indent or the entire first indent may comprise a plurality of perforations extending from the perimeter to the first end proximate the central region as described hereinabove. In any embodiment of any medical dressing disclosed herein, a portion of the second indent or the entire second indent may comprise a plurality of perforations extending from the perimeter to the second end proximate the central region as described hereinabove.

Referring to FIG. 5, the body 10 of the medical dressing 200 comprises a second flap 50. The second flap 50 is defined by a second distal end 52, a third indent 54 having a third end 55 proximate the central region 18, a fourth indent 56 having a fourth end 57 proximate the central region 18, and a second hinge region 58 extending between the third end 55 and the fourth end 57. The second distal end 52 is formed by a second part 16b of the perimeter 16 of the body 10. In any embodiment of the medical dressing 200, a portion of the third indent 54 or the entire third indent 54 and/or a portion of the fourth indent 56 or the entire fourth indent 56 may comprise a slit (i.e., a thin cut extending through the body 10).

In any embodiment (not shown) of a medical dressing disclosed herein, a portion of the third indent or the entire third indent may comprise a plurality of perforations extending from the perimeter to the third end proximate the central region. The perforations may comprise small holes and/or small slits generally aligned along a path to form the third indent. In any embodiment of any medical dressing disclosed herein, a portion of the fourth indent or the entire fourth indent may comprise a plurality of perforations extending from the perimeter to the fourth end proximate the central region. The perforations may comprise small holes and/or small slits generally aligned along a path to form the fourth indent. Advantageously, the perforations can be torn by the operator before, during, or after applying the dressing to the tubiform component or to the surface. Moreover, the perforated indent advantageously provides a visual cue that can be used by the operator to align the dressing with the tubiform component before and/or during the process of using the dressing to secure the tubiform component.

The body 10 of the medical dressing 200 further comprises a third lateral extension 60 extending in a first direction (not shown in FIG. 4) away from the second flap 50 and a fourth lateral extension 62 extending in a second direction (not shown in FIG. 5) away from the second flap 50, as described above for the first flap 20 medical dressing 200. The first direction is substantially opposite the second direction. In any embodiment of a medical dressing described herein, a lateral extension (e.g., first lateral extension 30 of FIG. 5) extending from the first flap in substantially the same direction as a lateral extension (e.g. third lateral extension 60 of FIG. 5) extending from the second flap optionally can be conjoined, as illustrated in FIG. 5. Similarly, in any embodiment of a medical dressing described herein, a lateral extension (e.g., second lateral extension 32 of FIG. 5) extending from the first flap in substantially the same direction as a lateral extension (e.g. fourth lateral extension 62 of FIG. 5) extending from the second flap optionally can be conjoined, as illustrated in FIG. 5.

The body 10 of the medical dressing 200 has similar properties and can be constructed of materials like those described hereinabove for the body 10 of the medical dressing 100. In addition, the medical dressing 200 further comprises an adhesive (not shown in FIG. 5) disposed on at least a portion of the second major surface of the body. The portion of the second major surface on which the adhesive is disposed includes at least a part of each of the first flap, the second flap, the first lateral extension, the second lateral extension, the third lateral extension, and the fourth lateral extension. The adhesive has similar properties and can be constructed of materials like those described hereinabove for the medical dressing 100. In certain alternative embodiments (not shown), the adhesive may be deposited (e.g., coated) onto the body using a process that results in a pattern of coated and uncoated areas on the second surface of the body. Thus, in some embodiments, the adhesive is disposed on about 10-100% of the second major surface. In some embodiments, the adhesive is disposed on less than 100% of the second major surface. In some embodiments, the adhesive is disposed on about 100% of the second major surface. Other aspects of the adhesive 40 of the medical device 100 are discussed elsewhere in the specification.

Optionally, in any embodiment, the medical dressing 200 can comprise at least one indent (e.g., fifth indent 44) extending from the perimeter 16 into or adjacent the first lateral extension 30. Advantageously, the fifth indent 44 provides greater conformability of the medical dressing 200 to an uneven surface and may reduce the likelihood of separation of the edge of the dressing from a skin surface when the underlying tissue (e.g., muscle tissue) is subjected to expansion or contraction. In any embodiment, a portion of the fifth indent 44 or the entire fifth indent 44 may comprise a slit (i.e., a thin cut extending through the body 10).

In any embodiment (not shown) of a medical dressing disclosed herein, a portion of the fifth indent or the entire fifth indent may comprise a plurality of perforations extending from the perimeter into the first lateral extension. The perforations may comprise small holes and/or small slits generally aligned along a path to form the fifth indent. Advantageously, the perforations can be torn by the operator before or during the application of the dressing to the tubiform component or to the surface.

In addition, in any embodiment, the medical dressing 100 optionally can comprise at least one indent (e.g., sixth indent 46) extending from the perimeter 16 into or adjacent the second lateral extension 32. Advantageously, the sixth indent 46 provides greater conformability of the medical dressing 100 to an uneven surface and may reduce the likelihood of separation of the edge of the dressing from a skin surface when the underlying tissue (e.g., muscle tissue) is subjected to expansion or contraction. In any embodiment, a portion of the sixth indent 46 or the entire sixth indent 46 may comprise a slit (i.e., a thin cut extending through the body 10).

In any embodiment of any medical dressing disclosed herein, a portion of the sixth indent or the entire sixth indent may comprise a plurality of perforations extending from the perimeter into the second lateral extension. The perforations may comprise small holes and/or small slits generally aligned along a path to form the sixth indent. Advantageously, the perforations can be torn by the operator before or during the application of the dressing to the tubiform component or to the surface.

In some embodiments, a perforated line (not shown) extends between the fifth indent and sixth indent. Advantageously, this feature aids in removal by allowing the device to be cleanly removed in two parts. Moreover, the fifth and sixth indents provide to the operator a visual cue showing where the perforation line is located. This feature (i.e., perforated line extending between the fifth and sixth indents) can be provided in any medical dressing according to the present disclosure.

The first flap 20 of the medical dressing 200 can define a first flap axis 29 extending from the perimeter 16 through the first hinge region 28. In addition, the second flap 50 of the medical dressing 200 can define a second flap axis 59 extending from the perimeter 16 through the second hinge region 58. In any embodiment of a medical dressing comprising first and second flaps according to the present disclosure, the first flap axis and the second flap axis optionally can be substantially aligned. It is contemplated within the invention that, in some embodiments, the first flap axis and the second flap axis can be offset to a small extent. In addition, it is contemplated that, in some embodiments, the first flap axis and the second flap axis, although located on opposite portions of the perimeter, optionally may not be parallel. Such minor deviations are within the scope of the invention.

In any embodiment, proximate the first end, the first indent of the body of the medical dressing 200, the first indent optionally comprises a portion (not shown in FIG. 5) that slants in a direction away from the first flap axis or that slants in a direction toward the first flap axis. Optionally, proximate the second end, the second indent of the body of the medical dressing 200 comprises a portion (not shown in FIG. 5) that slants in a direction away from the first flap axis or that slants in a direction toward the first flap axis, as described for the illustrated embodiment of FIG 2.

In any embodiment, proximate the third end, the third indent of the body of the medical dressing 200, the third indent optionally comprises a portion (not shown in FIG. 5) that slants in a direction away from the third flap axis or that slants in a direction toward the third flap axis. Optionally, proximate the fourth end, the fourth indent of the body of the medical dressing 200 comprises a portion (not shown in FIG. 5) that slants in a direction away from the second flap axis or that slants in a direction toward the second flap axis, in the manner as described for the first flap of the illustrated embodiment of FIG 2.

In any embodiment, the medical dressing 200 can comprise a body (not shown) having a first layer and a second layer as described above and shown in FIGS. 3A-B and 4A-C. In these embodiments, the second layer can overlap the first layer, or the first layer can overlap the second layer at third indent and the fourth indent of the second flap. The multilayer body can permit visualization of a portion of a surface covered by the medical dressing and/or provide support to prevent the formation of or reduce the extent of a tear propagating from the end (e.g., first end, second end, third end, and/or fourth end) of the indents forming the first flap or the second flap.

In any embodiment, the medical dressing 200 can comprise an optional liner (not shown in FIG. 5) as described above for the medical dressing 100. The liner covers all or a portion of the adhesive to prevent contamination of the adhesive. It is contemplated that the liner may comprise optional indents, perforations or slits (not shown) that optionally may be superimposed by first indent 24, second indent 26, third indent 54, and/or fourth indent 56 of the body 10.

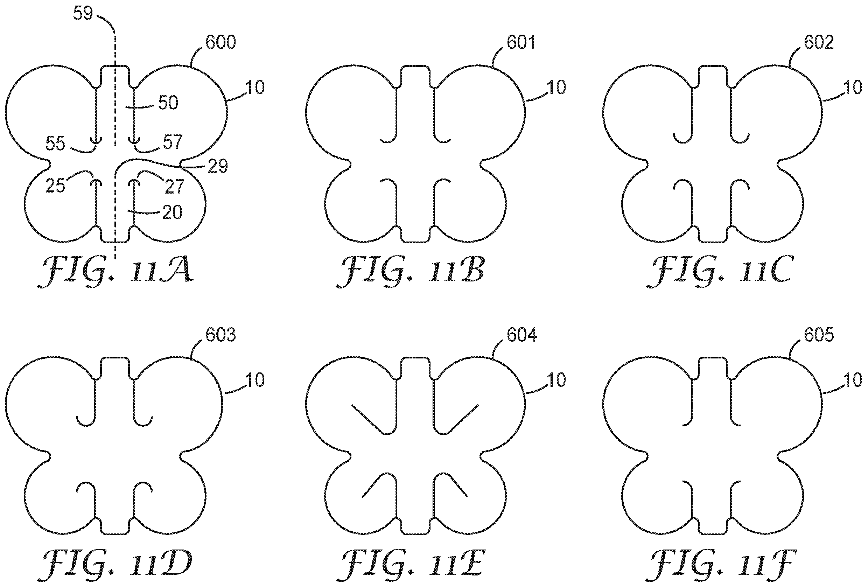

FIGS. 11A-F show six alternative embodiments (medical dressing 600, medical dressing 601, medical dressing 602, medical dressing 603, medical dressing 604, and medical dressing 605, respectively) of medical dressings each comprising a first flap 20 formed in part by a first indent 24 having a first end 25 and a second indent 26 having a second end 27, as shown in FIG 11A. The first flap 20 of each of the medical dressings comprises a first flap axis 29 and the second flap 50 of each of the medical dressings comprises a second flap axis 59 as shown in FIG. 11A. In each embodiment of the medical dressings, the first end comprises a portion that slants away from the first flap axis. In addition, in each embodiment of the medical dressings, the second end comprises a portion that slants away from the first flap axis. In addition, in each embodiment of the medical dressings, the third end comprises a portion that slants away from the second flap axis. In addition, in each embodiment of the medical dressings, the fourth end comprises a portion that slants away from the second flap axis.

In the illustrated embodiment of FIG. 11A, the first end 25 also comprises a portion that slants toward the first flap axis 29. In the illustrated embodiment of FIG. 11A, the second end 27 also comprises a portion that slants toward the first flap axis 29. In the illustrated embodiment of FIG. 11A, the third end 55 also comprises a portion that slants toward the second flap axis 59. In the illustrated embodiment of FIG. 11A, the fourth end 57 also comprises a portion that slants toward the second flap axis 59. The illustrated embodiments of FIGS. 11A-F represent non-limiting examples of features of the medical dressings of the present disclosure. These features confer certain advantages discussed herein.

In another aspect, the present disclosure provides a tape comprising a plurality of any of the medical dressings according to the present disclosure. The adhesive of each of the plurality of medical dressings is adhered to a unitary carrier. The unitary carrier can comprise, for example any embodiment of a liner disclosed herein. In certain embodiments, the unitary carrier may be rolled into a roll form. In certain alternative embodiments, the unitary carrier may be folded into a z-fold form. In any embodiment, the unitary carrier can comprise an area of weakness disposed between adjacent medical dressings. The area of weakness can facilitate tearing or otherwise separating at least one of the medical dressings disposed on the carrier from at least one other medical dressing disposed on the carrier.

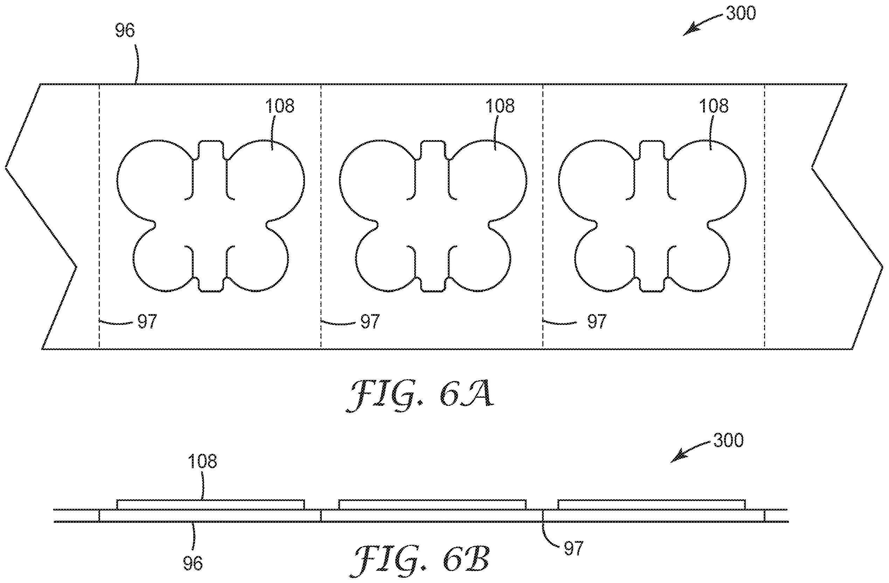

FIGS. 6A-B show various views of one embodiment of a tape 300 according to the present disclosure. The tape 300 comprises a unitary carrier 96 with a plurality of medical dressings 108 disposed thereon. The medical dressings 108 can be any embodiment of any medical dressing disclosed herein. The medical dressings 108 are releasably adhered to the carrier 96, either directly or indirectly, via the adhesive (not shown) disposed on the second major surface of the medical dressing

The unitary carrier 96 includes at least one area of weakness 97 disposed between adjacent medical dressings 108. The area of weakness facilitates separation (e.g., by tearing the unitary carrier), along a generally prescribed path, of at least one medical dressing from another medical dressing. The area of weakness can comprise, for example, a punch, a perforation, a cut, a slit, an indent, or a depression.

In any embodiment of the tape 96, the unitary carrier can be a protective liner as described herein. In one embodiment, a package that contains the plurality of adhesive medical dressings may serve as a unitary carrier.

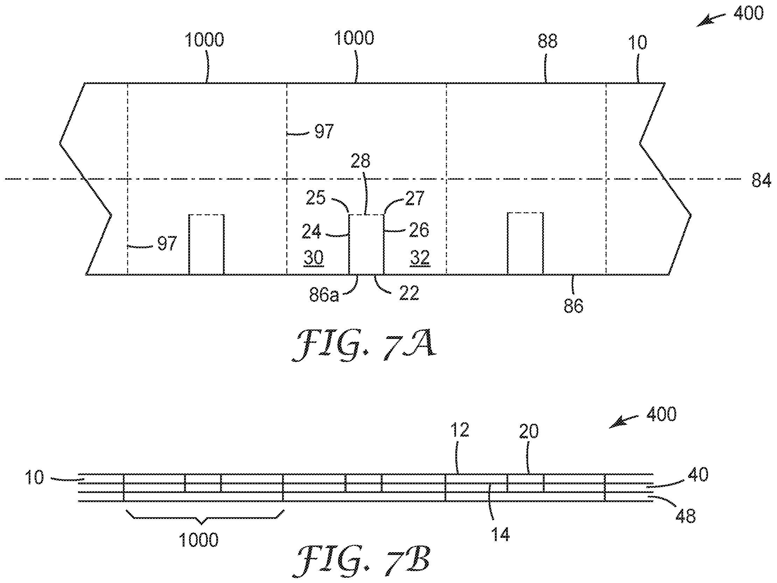

In yet another aspect, the present disclosure provides a medical tape comprising a body that comprises a plurality of body units, each body unit configured for securing a medical device comprising a tubiform component to a surface. FIGS. 7A and 7B show various views of one embodiment of a medical tape 400 according to the present disclosure.

The medical tape 400 comprises a body 10 that has a first major surface 12, a second major surface 14 opposite the first major surface 12, a first primary edge 86, a second primary edge 88, and a longitudinal axis 84.

The body 10 of the medical tape 400 can comprise a sheet material. Suitable sheet materials are well known in the art of medical dressings and include, but are not limited to, a transparent polymeric film, a translucent polymeric film, an opaque polymeric material, a woven fabric, a nonwoven fabric, a foam, or a combination of any two or more of the foregoing sheet materials. The material from which the body is constructed generally is flexible enough to conform to the shape of a tubiform component of a medical device and to a surface (e.g., a generally flat surface, an uneven surface) to which the medical device is to be attached via the medical tape. Other aspects of the body 10 of the medical tape are discussed elsewhere in the specification.

In some embodiments, the material from which the body 10 is constructed is flexible, but also has enough rigidity to be self-supporting. Advantageously, such materials (e.g., a relatively thick polymeric film; a laminate of a polymeric film and a woven material, a non-woven material, and/or a foam) are rigid enough to prevent the tape from unintentionally folding back on itself when applying the tape to a medical device.

The body 10 of the medical tape 400 further comprises a plurality of body units 1000. Each body unit 1000 comprises a first flap 20. The first flap 20 is defined by a first distal end 22, a first indent 24 having a first end 25 proximate the longitudinal axis 84, a second indent 26 having a second end 27 proximate the longitudinal axis, and a first hinge region 28 extending between the first end 25 and the second end 27. The first distal end 22 is formed by a first part 86a of the first primary edge 86. In any embodiment of a medical tape disclosed herein, a portion of the first indent 24 or the entire first indent 24 may comprise a slit (i.e., a thin cut extending through the body 10). In any embodiment of any medical dressing disclosed herein, a portion of the second indent 26 or the entire second indent 26 may comprise a slit (i.e., a thin cut extending through the body 10).

In any embodiment (not shown) of a medical tape disclosed herein, a portion of the first indent or the entire first indent may comprise a plurality of perforations extending from the first primary edge to the first end proximate the longitudinal axis. The perforations may comprise small holes and/or small slits generally aligned along a path to form the first indent. In any embodiment of any medical tape disclosed herein, a portion of the second indent or the entire second indent may comprise a plurality of perforations extending from the first primary edge to the second end proximate the longitudinal axis. The perforations may comprise small holes and/or small slits generally aligned along a path to form the second indent. Advantageously, the perforations can be torn by the operator before, during, or after applying the body unit to the tubiform component or to the surface. Moreover, the perforated indent advantageously provides a visual cue that can be used by the operator to align the body unit with the tubiform component before and/or during the process of using the body unit to secure the tubiform component.

Each body unit 1000 of the plurality of body units further comprises a first lateral extension 30 extending in a first direction away from the first flap 20 and a second lateral extension 32 extending in a second direction away from the first flap 20, as described above for the medical dressing 100. The first direction is substantially opposite the second direction.

The body 10 of the medical tape 400 has similar properties and can be constructed of materials like those described hereinabove for the body 10 of the medical dressing 100. In addition, the medical tape 400 further comprises an adhesive 40 disposed on at least a portion of the second major surface of the body. The portion of the second major surface on which the adhesive is disposed includes at least a part of each of the first flap, the second flap, the first lateral extension, and the second lateral extension of each body unit of the plurality of body units. The adhesive has similar properties and can be constructed of materials similar to those described hereinabove for the medical dressing 100.

Each of the body units 1000 of the plurality is joined to at least one adjacent body unit at an area of weakness 97 that extends through the body 10 substantially orthogonal to the longitudinal axis 84. The area of weakness 97 facilitates separation (e.g., by tearing the body), along a generally prescribed path, of at least one body unit 1000 from another body unit. The area of weakness can comprise, for example, a punch, a perforation, a cut, a slit, an indent, or a depression.

Also shown in FIG. 7B is an optional liner 48 as described above for the medical dressing 100. The liner 48 covers all or a portion of the adhesive 40 to prevent contamination of the adhesive. Other aspects of the adhesive 40 of the medical tape 400 are discussed elsewhere in the specification. It is contemplated that the liner 48 may comprise optional indents, perforations or slits (not shown) that optionally may be superimposed by first indent 24 and/or second indent 26 of the body 10.

In certain embodiments (not shown) of the medical tape of FIGS. 7A-B, the first flap defines a first flap axis extending from the perimeter through the first hinge region. In these embodiments, proximate the first end, the first indent comprises a portion that slants in a direction away from the first flap axis and/or a portion that slants in a direction toward the fist flap axis, as described hereinabove for the medical dressing 102.

In certain embodiments (not shown), the medical tape can comprise a body having a first layer and a second layer as described hereinabove. In these embodiments, the second layer can overlap the first layer, or the first layer can overlap the second layer at first indent and the second indent of the first flap. The multilayer body can permit visualization of a portion of a surface covered by the medical dressing and/or provide support to prevent the formation of or reduce the extent of a tear propagating from the end (e.g., first end and/or second end) of the indents forming the first flap. In certain embodiments (not shown), the second layer forms a frame structure that forms a border around a portion of the first layer and provides a transparent or translucent window in the dressing that permits observation through at least a part of the body proximate the longitudinal axis, as described herein above for the medical dressing 106.

The first layer defines a first area (not shown) that includes an area portion overlapping the second layer and optionally an area portion that does not overlap the second layer. The second layer defines a second area (not shown) that includes an area portion overlapped the second layer and optionally an area portion that is not overlapped by the first layer.

In certain embodiments (not shown) of a medical tape comprising a body that includes a first layer having a first area and a second layer having a second area as described herein, the first area can equal at least 50% of the second area, at least 60% of the second area, at least 70% of the second area, at least 80% of the second area, at least 90% of the second area, at least 95% of the second area, or about 100% of the second area. In certain embodiments (not shown) of a medical tape comprising a body that includes a first layer having a first area and a second layer having a second area as described herein, the first area can equal not more than 95% of the second area, not more than 90% of the second area, not more than 80% of the second area, not more than 70% of the second area, or not more than 60% of the second area.

In certain embodiments (not shown) of a medical tape comprising a body that includes a first layer and a second layer as described herein, the second area can equal at least 50% of the first area, at least 60% of the first area, at least 70% of the first area, at least 80% of the first area, at least 90% of the first area, at least 95% of the first area, or about 100% of the first area. In certain embodiments (not shown) of a medical tape comprising a body that includes a first layer and a second layer as described herein, the second area can equal not more than 95% of the first area, not more than 90% of the first area, not more than 80% of the first area, not more than 70% of the first area, or not more than 60% of the first area.

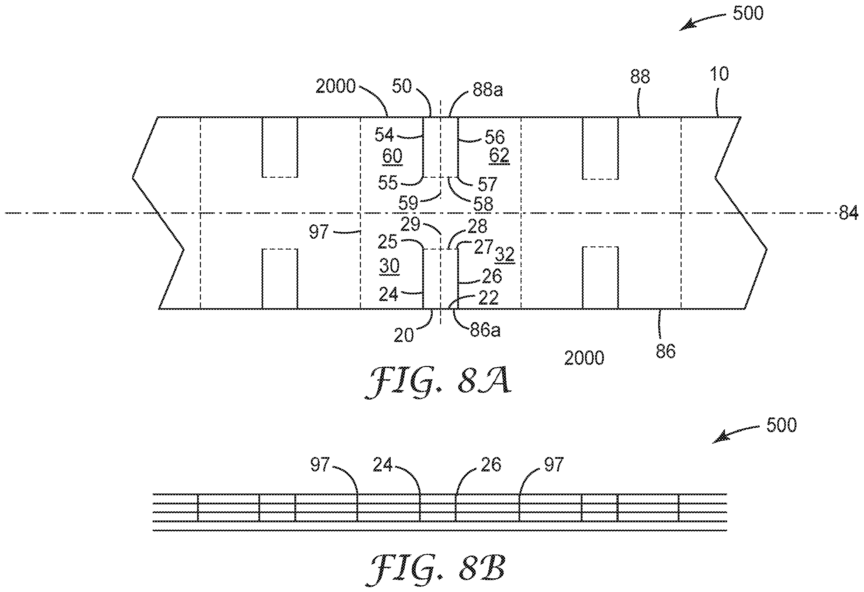

In any embodiment of a medical tape of the present disclosure, the body of the medical tape, in addition to comprising each and every element as set forth hereinabove for the medical tape 1000, further comprises a second flap. FIGS. 8A-B show various views of one embodiment of a medical tape 500 comprising a plurality of body units 2000, each body unit having a second flap 50.

The body 10 of the medical dressing 500 further comprises a plurality of body units 2000. Each body unit 2000 comprises a first flap 20. The first flap 20 is defined by a first distal end 22, a first indent 24 having a first end 25 proximate the longitudinal axis 84, a second indent 26 having a second end 27 proximate the longitudinal axis, and a first hinge region 28 extending between the first end 25 and the second end 27. The first distal end 22 is formed by a first part 86a of the first primary edge 86. In any embodiment of a medical tape disclosed herein, a portion of the first indent 24 or the entire first indent 24 may comprise a slit (i.e., a thin cut extending through the body 10). In any embodiment of any medical tape disclosed herein, a portion of the second indent 26 or the entire second indent 26 may comprise a slit (i.e., a thin cut extending through the body 10).

In any embodiment (not shown) of a medical tape disclosed herein, a portion of the first indent or the entire first indent may comprise a plurality of perforations extending from the first primary edge to the first end proximate the longitudinal axis as described hereinabove. In any embodiment of any medical tape disclosed herein, a portion of the second indent or the entire second indent may comprise a plurality of perforations extending from the first primary edge to the second end proximate the longitudinal axis as described hereinabove.

Each body unit 2000 of the plurality of body units further comprises a first lateral extension 30 extending in a first direction away from the first flap 20 and a second lateral extension 32 extending in a second direction away from the first flap 20, as described above for the medical dressing 100. The first direction is substantially opposite the second direction.

Each body unit 2000 further comprises a second flap 50. The second flap 50 is defined by a second distal end 52, a third indent 54 having a third end 55 proximate the longitudinal axis 84, a fourth indent 56 having a fourth end 57 proximate the longitudinal axis, and a second hinge region 58 extending between the third end 55 and the fourth end 57. The second distal end 52 is formed by a first part 88a of the second primary edge 88. In any embodiment of a medical tape disclosed herein, a portion of the third indent 54 or the entire third indent 54 may comprise a slit (i.e., a thin cut extending through the body 10). In any embodiment of any medical tape disclosed herein, a portion of the fourth indent 56 or the entire fourth indent 56 may comprise a slit (i.e., a thin cut extending through the body 10).

In any embodiment (not shown) of a medical tape disclosed herein, a portion of the third indent or the entire third indent may comprise a plurality of perforations extending from the second primary edge to the third end proximate the longitudinal axis as described hereinabove. In any embodiment of any medical tape disclosed herein, a portion of the fourth indent or the entire fourth indent may comprise a plurality of perforations extending from the perimeter to the fourth end proximate the longitudinal axis as described hereinabove.

Each body unit 2000 of the plurality of body units further comprises a third lateral extension 60 extending in a third direction away from the second flap 50 and a fourth lateral extension 62 extending in a fourth direction away from the second flap 50, as described above for the medical dressing 100. The third direction is substantially opposite the fourth direction.

The body 10 of the medical tape 500 has similar properties and can be constructed of materials like those described hereinabove for the body 10 of the medical tape 400. The medical tape 500 further comprises an adhesive 40 disposed on at least a portion of the second major surface of the body. The portion of the second major surface on which the adhesive is disposed includes at least a part of each of the first flap, the second flap, the first lateral extension, the second lateral extension, the third lateral extension, and the fourth lateral extension of each body unit of the plurality of body units.

The adhesive has similar properties and can be constructed of materials similar to those described hereinabove for the medical dressing 100. In certain alternative embodiments (not shown), the adhesive may be deposited (e.g., coated) onto the body using a process that results in a pattern of coated and uncoated areas on the second surface of the body. Thus, in some embodiments, the adhesive is disposed on about 10-100% of the second major surface. In some embodiments, the adhesive is disposed on less than 100% of the second major surface. In some embodiments, the adhesive is disposed on about 100% of the second major surface. Other aspects of the adhesive 40 of the medical device 100 are discussed elsewhere in the specification.

Each of the body units 2000 of the plurality is joined to at least one adjacent body unit at an area of weakness 97 that extends through the body 10 substantially orthogonal to the longitudinal axis 84. The area of weakness 97 facilitates separation (e.g., by tearing the body), along a generally prescribed path, of at least one body unit 2000 from another body unit. The area of weakness can comprise, for example, a punch, a perforation, a cut, a slit, an indent, or a depression.

Also shown in FIG. 8B is an optional liner 48 as described above for the medical tape 400. The liner 48 covers all or a portion of the adhesive 40 to prevent contamination of the adhesive. Other aspects of the adhesive 40 of the medical tape 500 are discussed elsewhere in the specification. It is contemplated that the liner 48 may comprise optional indents, perforations or slits (not shown) that optionally may be superimposed by first indent 24 and/or second indent 26 of the body 10.

In certain embodiments (not shown) of the medical tape of FIGS. 8A-B, the first flap defines a first flap axis extending from the perimeter through the first hinge region. In these embodiments, proximate the first end, the first indent comprises a portion that slants in a direction away from the first flap axis and/or a portion that slants in a direction toward the first flap axis, as described herein. In these embodiments, proximate the second end, the second indent optionally comprises a portion that slants in a direction away from the first flap axis and/or a portion that slants in a direction toward the first flap axis, as described herein. In these embodiments, proximate the third end, the third indent optionally comprises a portion that slants in a direction away from the second flap axis and/or a portion that slants in a direction toward the second flap axis, as described herein. In these embodiments, proximate the fourth end, the fourth indent optionally comprises a portion that slants in a direction away from the second flap axis and/or a portion that slants in a direction toward the second flap axis, as described herein.

In certain embodiments (not shown), the medical tape can comprise a body having a first layer and a second layer as described hereinabove. In these embodiments, the second layer can overlap the first layer, or the first layer can overlap the second layer at first indent and the second indent of the first flap. The multilayer body can permit visualization of a portion of a surface covered by the medical dressing and/or provide support to prevent the formation of or reduce the extent of a tear propagating from the end (e.g., first end and/or second end) of the indents forming the first flap. In certain embodiments (not shown), the second layer forms a frame structure that forms a border around a portion of the first layer and provides a transparent or translucent window in the dressing that permits observation through at least a part of the body proximate the longitudinal axis, as described herein above for the medical dressing 106.

The first layer defines a first area (not shown) that includes an area portion overlapping the second layer and optionally an area portion that does not overlap the second layer. The second layer defines a second area (not shown) that includes an area portion overlapped the second layer and optionally an area portion that is not overlapped by the first layer.

In certain embodiments (not shown) of a medical tape comprising a body that includes a first layer having a first area and a second layer having a second area as described herein, the first area can equal at least 50% of the second area, at least 60% of the second area, at least 70% of the second area, at least 80% of the second area, at least 90% of the second area, at least 95% of the second area, or about 100% of the second area. In certain embodiments (not shown) of a medical tape comprising a body that includes a first layer having a first area and a second layer having a second area as described herein, the first area can equal not more than 95% of the second area, not more than 90% of the second area, not more than 80% of the second area, not more than 70% of the second area, or not more than 60% of the second area.

In certain embodiments (not shown) of a medical tape comprising a body that includes a first layer and a second layer as described herein, the second area can equal at least 50% of the first area, at least 60% of the first area, at least 70% of the first area, at least 80% of the first area, at least 90% of the first area, at least 95% of the first area, or about 100% of the first area. In certain embodiments (not shown) of a medical tape comprising a body that includes a first layer and a second layer as described herein, the second area can equal not more than 95% of the first area, not more than 90% of the first area, not more than 80% of the first area, not more than 70% of the first area, or not more than 60% of the first area.

Referring to FIG. 8, each body unit 2000 of the medical tape 500 comprises a second flap 50. The second flap 50 is defined by a second distal end 52, a third indent 54 having a third end 55 proximate the central region 18, a fourth indent 56 having a fourth end 57 proximate the central region 18, and a second hinge region 58 extending between the third end 55 and the fourth end 57. The second distal end 52 is formed by a second part 88a of the second primary edge 88 of the body 10. In any embodiment of the medical tape 500, a portion of the third indent 54 or the entire third indent 54 and/or a portion of the fourth indent 56 or the entire fourth indent 56 may comprise a slit (i.e., a thin cut extending through the body 10).

In any embodiment (not shown) of a medical tape disclosed herein, a portion of the third indent or the entire third indent may comprise a plurality of perforations extending from the second primary edge to the third end proximate the longitudinal axis as described hereinabove. In any embodiment of any medical tape disclosed herein, a portion of the fourth indent or the entire fourth indent may comprise a plurality of perforations extending from the perimeter to the fourth end proximate the longitudinal axis as described hereinabove.

Each body unit 2000 of the body 10 further comprises a third lateral extension 60 extending in a first direction (not shown in FIG. 8) away from the second flap 50 and a fourth lateral extension 62 extending in a second direction (not shown in FIG. 8) away from the second flap 50, as described above for the first flap 20 medical dressing 200. The first direction is substantially opposite the second direction. In any embodiment of a medical tape described herein, a lateral extension (e.g., first lateral extension 30 of FIG. 8) extending from the first flap in substantially the same direction as a lateral extension (e.g. third lateral extension 60 of FIG. 8) extending from the second flap optionally can be conjoined, as illustrated in FIG. 8. Similarly, in any embodiment of a medical tape described herein, a lateral extension (e.g., second lateral extension 32 of FIG. 8) extending from the first flap in substantially the same direction as a lateral extension (e.g. fourth lateral extension 62 of FIG. 8) extending from the second flap optionally can be conjoined, as illustrated in FIG. 8.

The first flap 20 of each body unit 2000 of the medical tape 500 can define a first flap axis 29 extending from the perimeter 16 through the first hinge region 28. In addition, the second flap 50 of the medical dressing 200 can define a second flap axis 59 extending from the perimeter 16 through the second hinge region 58. In any embodiment of a medical dressing comprising first and second flaps according to the present disclosure, the first flap axis and the second flap axis optionally can be substantially aligned. It is contemplated within the invention that, in some embodiments, the first flap axis and the second flap axis can be offset to a small extent. In addition, it is contemplated that, in some embodiments, the first flap axis and the second flap axis, although located on opposite portions of the perimeter, optionally may not be parallel. Such minor deviations are within the scope of the invention.

### Systems for Securing a Medical Device to a Surface

In another aspect, the present disclosure provides a system for securing to a surface a medical device having a tubiform component. The system comprises the medical dressing of any embodiment according to the present disclosure and a medical device having a tubiform component, wherein the adhesive disposed on the second major surface of the first flap of the medical dressing contacts the tubiform component of the medical device. The medical devices include, but are not limited to, medical devices that comprise a catheter, a cannula, a medical tube (e.g., a drainage tube, a suction tube, a feeding tube, a respiratory tube), a wire, or a combination of any two or more of the foregoing medical devices. Optionally, wherein the medical dressing of the system comprises a second flap, the system further comprises the adhesive disposed on the second major surface of the second flap of the medical dressing contacting the tubiform component of the medical device.

In certain alternative embodiments, the system comprises any embodiment of the body unit of a medical tape according to the present disclosure and a medical device having a tubiform component, wherein the adhesive disposed on the second major surface of the first flap of the body unit contacts the tubiform component of the medical device. The medical devices include, but are not limited to, medical devices that comprise a catheter, a cannula, a medical tube (e.g., a drainage tube, a suction tube, a feeding tube, a respiratory tube), a wire, or a combination of any two or more of the foregoing medical devices. Optionally, wherein the body unit of the system comprises a second flap, the system further comprises the adhesive disposed on the second major surface of the second flap of the body unit contacting the tubiform component of the medical device.

### Methods of Securing a Medical Device to a Surface

In yet another aspect, the present disclosure provides methods of securing a medical device having a tubiform component to a receiving surface. In some embodiments, the receiving surface can be a surface of skin of a person or an animal. In some embodiments, the receiving surface can be a surface of an inanimate object such as, for example, a table, a cart, a wall, an instrument, a monitor, a pole, or a piece of clothing.

The first method comprises contacting an adhesive disposed on a second major surface of a first flap of a medical dressing with a first part of an outer surface of the tubiform component of the medical device. In any embodiment, contacting the adhesive with the first part can comprise applying manual pressure against the first flap to urge the adhesive against the first part of the outer surface of the tubiform component. Nonlimiting examples of medical dressings that are suitable for use in the first method include any embodiment of any medical dressing according to the present disclosure.

According to the first method, the medical dressing comprises a body comprising a first major surface; a second major surface opposite the first major surface; a perimeter; a central region; a first flap defined by a distal end formed by a first part of the perimeter, a first indent having a first end proximate the central region, a second indent having a second end proximate the central region, and a first hinge region extending between the first end and the second end; a first lateral extension extending in a first direction away from the first flap; and a second lateral extension extending in a second direction away from the first flap. The medical dressing further comprises an adhesive disposed on at least a portion of the second major surface, wherein the portion of the second major surface on which the adhesive is disposed includes a part of each of the first flap, the first lateral extension, and the second lateral extension.

The first method further comprises contacting the adhesive disposed on the second major surface of the first lateral extension of the medical dressing with a first part of the receiving surface and contacting the adhesive disposed on the second major surface of the second lateral extension of the medical dressing with a second part of the receiving surface. In any embodiment, contacting the adhesive with the first part of the receiving surface can comprise applying manual pressure against the first lateral extension to urge the adhesive against the first part of the receiving surface. In any embodiment, contacting the adhesive with the second part of the receiving surface can comprise applying manual pressure against the second lateral extension to urge the adhesive against the second part of the receiving surface.

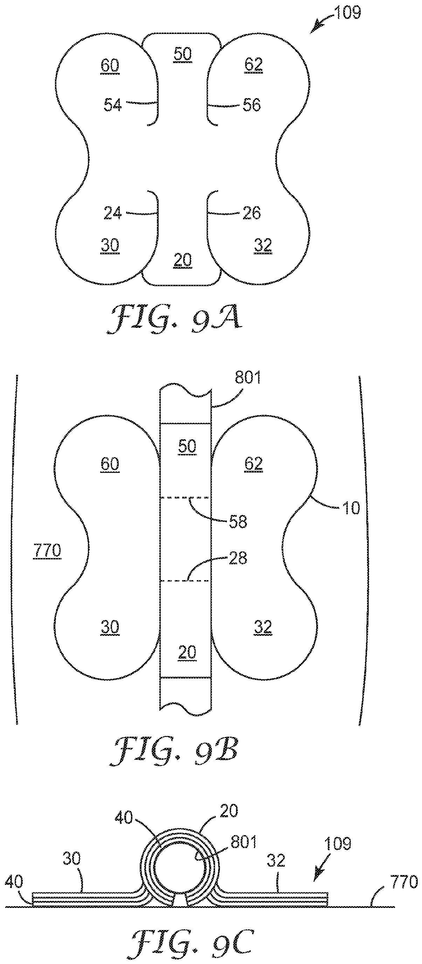

In any embodiment of the first method, contacting the adhesive disposed on the second major surface of the first flap with the first part of the outer surface of the tubiform component of the medical device further comprises wrapping the first flap around the outer surface of the tubiform component, as shown FIG. 9B. The outer surface of the tubiform component has a circumference (not shown). Wrapping the first flap around the first part of the outer surface of the tubiform component can comprise wrapping the first flap around at least 10%, at least 20%, at least 30%, at least 40%, at least 50%, at least 60%, at least 70%, at least 80%, at least 90%, or at least 95% of the circumference. In certain embodiments, wrapping the first flap around the first part of the outer surface of the tubiform component can comprise wrapping the first flap around 100% of the circumference.

Before contacting the adhesive disposed on the second major surface of the first flap of the medical dressing with the first part of the outer surface of the tubiform component and/or before contacting the adhesive disposed on the second major surface of the first lateral extension and/or the second lateral extension with the surface to which the tubiform component is to be secured, the first method further can comprise removing a liner from at least a part of the adhesive disposed on the second major surface of the body.

In certain embodiments of the first method, the body of the medical dressing further comprises a second flap defined by a distal end formed by a second part of the perimeter, a third indent having a third end proximate the central region, a fourth indent having a fourth end proximate the central region, and a second hinge region extending between the third end and the fourth end; a third lateral extension extending in a third direction away from the second flap; and a fourth lateral extension extending in a fourth direction away from the second flap. The third direction is substantially opposite the fourth direction. The first part of the perimeter is located on the perimeter opposite the second part of the perimeter. In these embodiments, the first method further comprises contacting the adhesive disposed on the second major surface of the second flap with a second part of the outer surface of the tubiform component, contacting the adhesive disposed on the second major surface of the third lateral extension with a third part of the receiving surface, contacting the adhesive disposed on the second major surface of the fourth lateral extension with a fourth part of the receiving surface.

In the embodiments of the first method wherein the body of the medical dressing further comprises a second flap, contacting the adhesive disposed on the second major surface of the second flap with the second part of the outer surface of the tubiform component can comprise applying manual pressure to a part of the first major surface of the second flap. In addition, contacting the adhesive disposed on the second major surface of the third lateral extension with a third part of the receiving surface can comprise applying manual pressure to a part of the first major surface of the third lateral extension and contacting the adhesive disposed on the second major surface of the fourth lateral extension with a fourth part of the receiving surface can comprise applying manual pressure to a part of the first major surface of the fourth lateral extension.

In any embodiment of the first method, contacting the adhesive disposed on the second major surface of the second flap with the outer surface of the tubiform component of the medical device further comprises wrapping the second flap around the second part of the outer surface of the tubiform component. The outer surface of the tubiform component has a circumference (not shown). Wrapping the second flap around the second part of the outer surface of the tubiform component can comprise wrapping the second flap around at least 10%, at least 20%, at least 30%, at least 40%, at least 50%, at least 60%, at least 70%, at least 80%, at least 90%, or at least 95% of the circumference. In certain embodiments, wrapping the second flap around the second part of the outer surface of the tubiform component can comprise wrapping the second flap 100% of the circumference.

Before contacting the adhesive disposed on the second major surface of the second flap of the medical dressing with the second part of the outer surface of the tubiform component and/or before contacting the adhesive disposed on the second major surface of the third lateral extension and/or the fourth lateral extension with the surface to which the tubiform component is to be secured, the first method further can comprise removing a liner from at least a part of the adhesive disposed on the second major surface of the body.

The present disclosure provides a second method of securing a medical device having a tubiform component to a receiving surface. In some embodiments, the receiving surface can be a surface of skin of a person or an animal. In some embodiments, the receiving surface can be a surface of an inanimate object such as, for example, a table, a cart, a wall, an instrument, a monitor, a pole, or a piece of clothing.

The second method comprises contacting an adhesive disposed on a second major surface of a first flap of a body unit of a medical tape with a first part of an outer surface of the tubiform component of the medical device. In any embodiment, contacting the adhesive with the first part can comprise applying manual pressure against the first flap to urge the adhesive against the first part of the outer surface of the tubiform component. Nonlimiting examples of medical tapes that are suitable for use in the first method include any embodiment of any medical tape according to the present disclosure. The medical tape comprises a plurality of body units, each body unit detachably attached to an adjacent body unit.

According to the second method the body unit comprises a first major surface; a second major surface opposite the first major surface; a first primary edge; a second primary edge; a longitudinal axis; a first flap defined by a distal end formed by a part of the first primary edge or a part of the secondary edge, a first indent having a first end proximate the longitudinal axis, a second indent having a second end proximate the longitudinal axis, and a first hinge region extending between the first end and the second end; a first lateral extension extending in a first direction away from the first flap; a second lateral extension extending in a second direction away from the first flap, wherein the first direction is substantially opposite the second direction; and an adhesive disposed on at least a portion of the second major surface. The portion of the second major surface on which the adhesive is disposed includes a part of each of the first flap, the first lateral extension, and the second lateral extension.

The second method further comprises contacting the adhesive disposed on the second major surface of the first lateral extension of the medical dressing with a first part of the receiving surface and contacting the adhesive disposed on the second major surface of the second lateral extension of the medical dressing with a second part of the receiving surface. In any embodiment, contacting the adhesive with the first part of the receiving surface can comprise applying manual pressure against the first lateral extension to urge the adhesive against the first part of the receiving surface. In any embodiment, contacting the adhesive with the second part of the receiving surface can comprise applying manual pressure against the second lateral extension to urge the adhesive against the second part of the receiving surface.

In any embodiment of the second method, contacting the adhesive disposed on the second major surface of the first flap with the first part of the outer surface of the tubiform component of the medical device further comprises wrapping the first flap around the outer surface of the tubiform component. The outer surface of the tubiform component has a circumference (not shown). Wrapping the first flap around the first part of the outer surface of the tubiform component can comprise wrapping the first flap around at least 10%, at least 20%, at least 30%, at least 40%, at least 50%, at least 60%, at least 70%, at least 80%, at least 90%, or at least 95% of the circumference. In certain embodiments, wrapping the first flap around the first part of the outer surface of the tubiform component can comprise wrapping the first flap around 100% of the circumference.

Before contacting the adhesive disposed on the second major surface of the first flap of the body unit with the first part of the outer surface of the tubiform component and/or before contacting the adhesive disposed on the second major surface of the first lateral extension and/or the second lateral extension with the surface to which the tubiform component is to be secured, the second method further can comprise removing a liner from at least a part of the adhesive disposed on the second major surface of the body unit.

In certain embodiments of the second method, the body unit further comprises a second flap defined by a distal end formed by a second part of the second primary edge, a third indent having a third end proximate the longitudinal axis, a fourth indent having a fourth end proximate the longitudinal axis, and a second hinge region extending between the third end and the fourth end; a third lateral extension extending in a third direction away from the second flap; and a fourth lateral extension extending in a fourth direction away from the second flap. The third direction is substantially opposite the fourth direction. The first part of the first primary edge is located on the perimeter opposite the second part of the second primary edge. In these embodiments, the second method further comprises contacting the adhesive disposed on the second major surface of the second flap with a second part of the outer surface of the tubiform component, contacting the adhesive disposed on the second major surface of the third lateral extension with a third part of the receiving surface, and contacting the adhesive disposed on the second major surface of the fourth lateral extension with a fourth part of the receiving surface.

In the embodiments of the second method wherein the body unit further comprises a second flap, contacting the adhesive disposed on the second major surface of the second flap with the second part of the outer surface of the tubiform component can comprise applying manual pressure to the first major surface of the second flap. In addition, contacting the adhesive disposed on the second major surface of the third lateral extension with a third part of the receiving surface can comprise applying manual pressure to the first major surface of the third lateral extension and contacting the adhesive disposed on the second major surface of the fourth lateral extension with a fourth part of the receiving surface can comprise applying manual pressure to the first major surface of the fourth lateral extension.

In any embodiment of the second method, contacting the adhesive disposed on the second major surface of the second flap with the outer surface of the tubiform component of the medical device further comprises wrapping the second flap around the second part of the outer surface of the tubiform component. The outer surface of the tubiform component has a circumference (not shown). Wrapping the second flap around the second part of the outer surface of the tubiform component can comprise wrapping the second flap around at least 10%, at least 20%, at least 30%, at least 40%, at least 50%, at least 60%, at least 70%, at least 80%, at least 90%, or at least 95% of the circumference. In certain embodiments, wrapping the second flap around the second part of the outer surface of the tubiform component can comprise wrapping the second flap around 100% of the circumference.

Before contacting the adhesive disposed on the second major surface of the second flap of the medical dressing with the second part of the outer surface of the tubiform component and/or before contacting the adhesive disposed on the second major surface of the third lateral extension and/or the fourth lateral extension with the surface to which the tubiform component is to be secured, the second method further can comprise removing a liner from at least a part of the adhesive disposed on the second major surface of the body.

Turning back to the drawings, FIG. 9A shows a plan view of one embodiment of a medical dressing 109 according to the present disclosure. Accordingly, the medical dressing 109 includes a body 10 with a first flap 20 flanked by a first indent 24 and a second indent 26, a second flap 50 flanked by a third indent 54 and a second indent 56, a first lateral extension 30, a second lateral extension 32, a third lateral extension 60, and a fourth lateral extension 62.

FIG. 9B shows a plan view of the medical dressing 109 securing a tubiform component 801 of a medical device (e.g., a catheter) to a surface 770 (e.g., a surface of skin). The first flap 20 and second flap 50 are adhered to the tubiform component 801 and the first lateral extension 30, second lateral extension 32, third lateral extension 60, and fourth lateral extension 62 each are adhered to the surface 770. Also shown in FIG. 9B are the first hinge region 28 and second hinge region 58 of the medical dressing 109.

FIG. 9C shows an end view of the medical dressing 109 and tubiform component 801 adhered to the surface 770 of FIG. 9B. The multi-layer first lateral extension 30 and second lateral extension 32 of the body 10 are shown adhered to the surface 770 via the adhesive 40 and the multi-layer first flap 20 is shown adhered to the tubiform component 801 via the adhesive 40. The first flap 20 is wrapped around almost 100% of the circumference of the tubiform component 801.

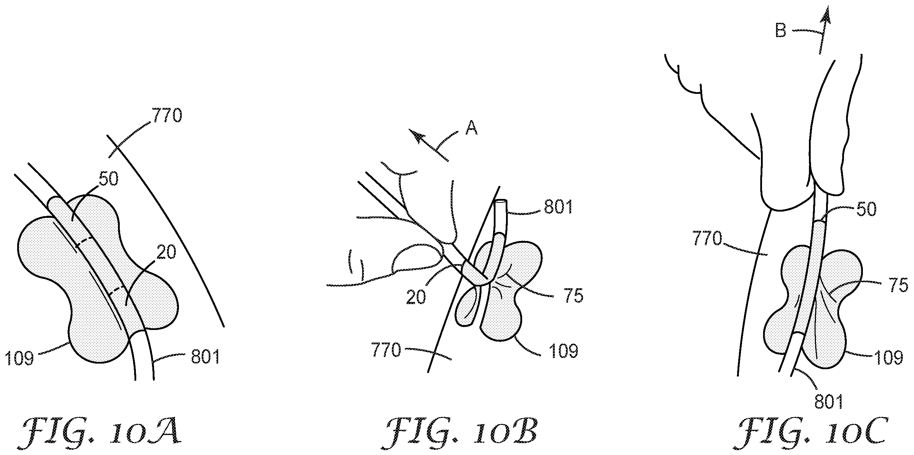

FIG. 10A shows an embodiment of a medical dressing 109 adhered to a surface 770 (e.g., a surface of skin). A tubiform component 801 of a medical device (e.g., a catheter) is overlaid by the medical dressing 109. The first flap 20 and the second flap 50 are wrapped around the tubiform component according to the method of the present disclosure.

FIG. 10B shows what happens to the medical dressing 109 of FIG. 10 when the tubiform component 801 is pulled in a direction "A" that is orthogonal to the plane of the surface 770 (e.g., away from a skin surface). The first flap 20 of the medical dressing 109 moves away from the surface 770 with the tubiform component 801 by flexing at the hinge region (not shown). In addition, the stress of the upward force is dispersed and counteracted by the adhesion of a broad area of the central region of the dressing (not shown in FIG. 10B), as evidenced by stress lines 75 that appear as wrinkles in the dressing. If a similar dressing (not shown) without a first flap 20 was subjected to the same force, all of the force would be directed against the edge of the dressing and would result in significant loss of adhesion of the edge of the dressing, if not the entire dressing. Without being bound by the aforementioned theory, it is an advantage of the medical dressings and body units of the medical tapes of the present disclosure that they remain attached to the surface when subjected to forces that would otherwise result in at least the loss of edge adhesion by dressings lacking the first or second flap when subjected to the same force against the tubiform component.

FIG. 10C shows what happens to the medical dressing 109 of FIG. 10 when the tubiform component 801 is pulled in a direction "B" in an axial direction of the tubiform component along the plane of the surface 770. The second flap 50 of the medical dressing 109 stretches somewhat along the axial direction of the force. In addition, the stress of the force is dispersed and counteracted by the adhesion of a broad area of the central region of the dressing (not shown in FIG. 10C), as evidenced by stress lines 75 that appear as wrinkles in the dressing. If a similar dressing (not shown) without a second flap 50 was subjected to the same force, all of the force would be directed against the edge of the dressing and would result in significant loss of adhesion of the edge of the dressing, if not the entire dressing. Without being bound by the aforementioned theory, it is a further advantage of the medical dressings and body units of the medical tapes of the present disclosure that they remain attached to the surface when subjected to similar forces that would otherwise result in at least the loss of edge adhesion by dressings lacking the first or second flap when subjected to the same force against the tubiform component.

### Body

The medical dressings and tapes are useful to provide securement of a medical device to a surface. Representative sheet materials for forming the body of the medical dressing or medical tape may include non-woven and woven fibrous webs, knits, films, foams polymeric films and other familiar backing materials. In some embodiments, a transparent sheet material is desirable to allow for viewing of the underlying skin or medical device.

In some embodiments, the construction of the device may be a multilayer laminate. The device described herein may be made by conventional techniques (e.g., extrusion, solvent casting, calendaring, laminating, adhesive coating, and the like) which are familiar to those skilled in the art. U.S. Pat. No. 6,685,682 discloses some potentially useful constructions and methods for making medical dressings with backing layers and support material as described herein..

The first layer is preferably a high moisture vapor permeable film such as described in U.S. Pat. Nos. 3,645,835 and 4,595,001. In one embodiment, the first layer is comprised of an elastomeric polyurethane, polyester, or polyether block amide films. These films combine the desirable properties of resiliency, elasticity, high moisture vapor permeability, and transparency. A description of this characteristic of materials for constructing the first layer can be found in issued U.S. Pat. Nos. 5,088,483 and 5,160,315. Commercially available examples of potentially suitable sheet materials for the first layer may include the thin polymeric film backings sold under the trade names TEGADERM (3M Company), OPSITE (Smith & Nephew), etc. Many other first layer materials may also be used, including those commonly used in the manufacture of surgical incise drapes (e.g., incise drapes manufactured by 3M Company under the trade names STERIDRAPE and IOBAN), etc.

The aforementioned first layer can be laminated to a second layer by means of an adhesive layer. The second layer materials used in one or more embodiments of medical dressings as described herein may provide strength to the backing layer. The second material therefore has more stiffness and less elasticity than the backing layer. The second material may be a coating, such as an adhesive, or may be a self-supporting substrate such as another film, woven, knitted, or nonwoven fabric. For example, U.S. Pat. No. 5,088,483 discloses a permanent adhesive as a reinforcement that could be used as the second material. One example of nonwoven for the second material is a high strength nonwoven fabric available from E. I. Dupont de Nemours & Company of Wilmington, Del. under the trademark Sontara, including Sontara 8010, a hydroentangled polyester fabric. Other suitable nonwoven webs include a hydroentangled polyester fabric available from Veratec, a division of International Paper of Walpole, Mass. Another suitable nonwoven web is the nonwoven elastomeric web described in U.S. Pat. No. 5,230,701.

It may be desirable that the first layer be kept relatively thin to, e.g., improve conformability. For example, the first layer may be formed of polymeric films with a thickness of 200 micrometers or less, or 100 micrometers or less, potentially 50 micrometers or less, or even 25 micrometers or less.

The first layer can be laminated to a second layer by means of an adhesive layer. The second-layer materials used in one or more embodiments of medical dressings as described herein may provide strength to the first layer. The second-layer material therefore has more stiffness and less elasticity than the first layer. The second-layer material may be a coating, such as an adhesive, or may be a self-supporting substrate such as another film, woven, knitted, or nonwoven fabric. For example, U.S. Pat. No. 5,088,483 discloses a permanent adhesive as a reinforcement that could be used as a support material. One example of nonwoven for the second-layer material is a high strength nonwoven fabric available from E. I. Dupont de Nemours & Company of Wilmington, Del. under the trademark Sontara, including Sontara 8010, a hydroentangled polyester fabric. Other suitable nonwoven webs include a hydroentangled polyester fabric available from Veratec, a division of International Paper of Walpole, Mass. Another suitable nonwoven web is the nonwoven elastomeric web described in U.S. Pat. No. 5,230,701.

Suitable adhesives for use in wound dressing articles include any adhesive that provides acceptable adhesion to skin and is acceptable for use on skin (e.g., the adhesive should preferably be nonirritating and non-sensitizing). Suitable adhesives are pressure sensitive and in certain embodiments have a relatively high moisture vapor transmission rate to allow for moisture evaporation. Suitable pressure sensitive adhesives include those based on acrylates, urethane, hydrogels, hydrocolloids, block copolymers, silicones, rubber-based adhesives (including natural rubber, polyisoprene, polyisobutylene, butyl rubber etc.) as well as combinations of these adhesives. The adhesive component may contain tackifiers, plasticizers, rheology modifiers as well as active components. Inclusion of medicaments or antimicrobial agents in the adhesive is also contemplated, as described in U.S. Pat. Nos. 4,310,509 and 4,323,557. Silicone adhesive can also be used. Generally, silicone adhesives can provide suitable adhesion to skin while gently removing from skin. Suitable silicone adhesives are disclosed in PCT Publications WO2010/056541 and WO2010/056543.

The pressure sensitive adhesives that may be used in the wound dressings may include adhesives that are typically applied to the skin such as the acrylate copolymers described in U.S. Patent No. RE 24,906, particularly a 97:3 isooctyl acrylate:acrylamide copolymer. Another example may include a 70: 15: 15 isooctyl acrylate: ethyleneoxide acrylate: acrylic acid terpolymer, as described in U.S. Patent No. 4,737,410 (Example 31). Other potentially useful adhesives are described in U.S. Patent Nos. 3,389,827; 4,112,213; 4,310,509; and 4,323,557. Inclusion of medicaments or antimicrobial agents in the adhesive is also contemplated, as described in U.S. Patent Nos. 4,310,509 and 4,323,557.

The pressure sensitive adhesives may, in some embodiments, transmit moisture vapor at a rate greater to or equal to that of human skin. While such a characteristic can be achieved through the 10 selection of an appropriate adhesive, it is also contemplated that other methods of achieving a high relative rate of moisture vapor transmission may be used, such as pattern coating the adhesive on the backing, as described in U.S. Patent No. 4,595,001. Other potentially suitable pressure sensitive adhesives may include blown-micro-fiber (BMF) adhesives such as, for example, those described in U.S. Patent No. 6,994,904. The pressure sensitive adhesive used in the wound dressing may also include one or more areas in which the adhesive itself includes structures such as, e.g., the microreplicated structures described in U.S. Patent No. 6,893,655.

Issued U.S. Patent Nos. 3,645,835 and 4,595,001 describe methods of making such films and methods for testing their permeability. Preferably, the film/adhesive composite should transmit moisture vapor at a rate equal to or greater than human skin. Preferably, the adhesive coated film transmits moisture vapor at a rate of at least 300 g/m2/24 hrs/37 C/100-10% RH, more preferably at least 700 g/m2/24 hrs/37 C/100-10% RH, and most preferably at least 2000 g/m2/24 hrs/37 C/100-10% RH using the inverted cup method as described in U.S. Patent No. 4,595,001.

Different portions (e.g., first flap, second flap, first lateral extension, second lateral extension, etc.) of the dressing may include different adhesives, such as disclosed in U.S. Patent Application 61/664,246 filed June 26, 2012 titled "Medical Dressing with Multiple Adhesives." For example, a portion may include an acrylate adhesive while another portion may include a silicone adhesive. In one embodiment, to prevent edge separation, adjacent the perimeter is acrylate adhesive, while near the central portion there is silicone adhesive. In one embodiment, to strongly secure with a device or tubing near the central portion there is acrylate adhesive, while near the perimeter in contact with skin is silicone adhesive.

In certain embodiments, the adhesive is coated (e.g., uniformly coated) along the entire second major surface of the body of any medical dressing or medical tape according to the present disclosure. In certain embodiments (not shown), the adhesive may be deposited (e.g., coated) onto the body using a process that results in a pattern of coated and uncoated areas on the second surface of the body. Thus, in some embodiments, the adhesive is disposed on about 10-100% of the second major surface. In some embodiments, the adhesive is disposed on less than 100% of the second major surface. In some embodiments, the adhesive is disposed on about 100% of the second major surface.

### Optional Components

The optional liner, when present, covers all or a portion of the adhesives to prevent contamination of the adhesives. In one embodiment, a package that contains the adhesive dressing may serve as a release liner. Suitable liners can be made of kraft papers, polyethylene, polypropylene, polyester or composites of any of these materials. In one embodiment, the liners are coated with release agents such as fluorochemicals or silicones. For example, U.S. Pat. No. 4,472,480 describes low surface energy perfluorochemical liners. In one embodiment, the liners are papers, polyolefin films, or polyester films coated with silicone release materials.

In certain alternative embodiments, the optional liner may include a microstructured surface instead of, or in addition to, a coated release agent. Nonlimiting examples of suitable microstructured liners are described in, for example, International Publication No. WO 2016/200685.

## Claims

1. A medical dressing (100, 102, 104, 106, 108, 109, 200, 600, 601. 602, 603, 604, 605), comprising:
a body (10) comprising:
a first major surface (12);
a second major surface (14) opposite the first major surface;
a perimeter (16);
a central region (18);
a first flap (20) defined by a first distal end (22) formed by a first part (16a) of the perimeter, a first indent (24) having a first end (25) proximate the central region, a second indent (26) having a second end (27) proximate the central region, and a first hinge region (28) extending between the first end and the second end;
wherein the first flap has a maximum length (y) and a maximum width (x);
wherein a ratio (r) of the maximum width to the maximum length is less than 4 to 1;
a first lateral extension (30) extending in a first direction (S) away from the first flap; and
a second lateral extension (32) extending in a second direction (T) away from the first flap;
wherein the first direction is substantially opposite the second direction;
an adhesive (40) disposed on at least a portion of the second major surface;
wherein the portion of the second major surface on which the adhesive is disposed includes a part of each of the first flap, the first lateral extension, and the second lateral extension.

2. The medical dressing of claim 1:
wherein the first flap (20) defines a first flap axis (29) extending from the perimeter (16) through the first hinge region (28);
wherein, proximate the first end (25), the first indent (24) comprises a portion (124) that extends in a direction away from the first flap axis.

3. The medical dressing of claim 1 or claim 2:
wherein the first flap (20) defines a first flap axis (29) extending from the perimeter (16) through the first hinge region (28);
wherein, proximate the second end (27), the second indent (26) comprises a portion (126) that extends in a direction away from the first flap axis.

4. The medical dressing of any one of the preceding claims, wherein the body (10) comprises sheet material selected from the group consisting of a transparent polymeric film, a translucent polymeric film, an opaque polymeric film, a woven fabric, a nonwoven fabric, a foam, and a combination of any two or more of the foregoing sheet materials.

5. The medical dressing of claim 4, wherein the sheet material is self-supporting.

6. The medical dressing of claim 4, wherein at least a portion (10a) of the body (10) comprises a first layer (98) overlaying and bonded to a second layer (99).

7. The medical dressing of claim 6, wherein the portion (10a) of the body (10) includes the first indent (24) and the second indent (26).

8. The medical dressing of claim 6 or claim 7, wherein the portion (10a) of the body (10) forms a frame structure that forms a border around a portion of the first layer (98) and provides a transparent or translucent window in the dressing that permits observation of at least a portion of the central region (18).

9. The medical dressing of any one of claims 6 through 8, wherein the first layer (98) defines a first area, wherein the second layer (99) defines a second area, wherein the first area is at least 50% of the second area.

10. The medical dressing of claim 9, wherein the first area is at least 90% of the second area.

11. The medical dressing of any one of claims 6 through 8, wherein the first layer (98) defines a first area, wherein the second layer (99) defines a second area, wherein the second area is at least 50% of the first area.

12. The medical dressing of any one of claims 6 through 8, wherein the first layer (98) defines a first area, wherein the second layer (99) defines a second area, wherein the second area is at least 90% of the first area.

13. The medical dressing of any one of the preceding claims, wherein the first indent (24) comprises a first slit portion.

14. The medical dressing of any one of the preceding claims, wherein the second indent (26) comprises a second slit portion.

15. The medical dressing of any one of the preceding claims, wherein the body (10) further comprises a fifth indent (44) extending from the perimeter (16) into or adjacent the first lateral extension (30).

## Patentansprüche

1. Ein medizinischer Verband (100, 102, 104, 106, 108, 109, 200, 600, 601, 602, 603, 604, 605), aufweisend:
einen Körper (10), aufweisend:
eine erste Hauptoberfläche (12);
eine zweite Hauptoberfläche (14) gegenüber der ersten Hauptoberfläche;
einen Umfang (16);
einen zentralen Bereich (18);
eine erste Lasche (20), die durch ein erstes distales Ende (22) definiert ist, das durch einen ersten Teil (16a) des Umfangs gebildet ist, einen ersten Einschnitt (24), der ein erstes Ende (25) nahe dem zentralen Bereich aufweist, einen zweiten Einschnitt (26), der ein zweites Ende (27) nahe dem zentralen Bereich aufweist, und einen ersten Falzbereich (28), der sich zwischen dem ersten Ende und dem zweiten Ende erstreckt;
wobei die erste Lasche eine maximale Länge (y) und eine maximale Breite (x) aufweist;
wobei ein Verhältnis (r) der maximalen Breite zu der maximalen Länge weniger als 4 bis 1 beträgt;
eine erste seitliche Erstreckung (30), die sich in einer ersten Richtung (S) von der ersten Lasche weg erstreckt; und
eine zweite seitliche Erstreckung (32), die sich in einer zweiten Richtung (T) von der ersten Lasche weg erstreckt;
wobei die erste Richtung im Wesentlichen entgegengesetzt zur zweiten Richtung ist;
einen Klebstoff (40), der auf mindestens einem Abschnitt der zweiten Hauptoberfläche angeordnet ist;
wobei der Abschnitt der zweiten Hauptoberfläche, auf dem der Klebstoff angeordnet ist, einen Teil jeder der ersten Lasche, der ersten seitlichen Erstreckung und der zweiten seitlichen Erstreckung enthält.

2. Der medizinische Verband nach Anspruch 1:
wobei die erste Lasche (20) eine erste Laschenachse (29) definiert, die sich von dem Umfang (16) durch den ersten Falzbereich (28) erstreckt;
wobei nahe dem ersten Ende (25) der erste Einschnitt (24) einen Abschnitt (124) aufweist, der sich in einer Richtung weg von der ersten Laschenachse erstreckt.

3. Der medizinische Verband nach Anspruch 1 oder 2:
wobei die erste Lasche (20) eine erste Laschenachse (29) definiert, die sich von dem Umfang (16) durch den ersten Falzbereich (28) erstreckt;
wobei nahe dem zweiten Ende (27) der zweite Einschnitt (26) einen Abschnitt (126) aufweist, der sich in einer Richtung weg von der ersten Laschenachse erstreckt.

4. Der medizinische Verband nach einem der vorstehenden Ansprüche, wobei der Körper (10) ein Lagenmaterial aufweist, das aus der Gruppe ausgewählt ist, die aus einer transparenten Polymerfolie, einer durchscheinenden Polymerfolie, einer opaken Polymerfolie, einem gewebten Stoff, einem Vliesstoff, einem Schaum und einer Kombination von beliebigen zwei oder mehr der vorstehenden Lagenmaterialien besteht.

5. Der medizinische Verband nach Anspruch 4, wobei das Lagenmaterial selbsttragend ist.

6. Der medizinische Verband nach Anspruch 4, wobei mindestens ein Abschnitt (10a) des Körpers (10) eine erste Schicht (98) aufweist, die eine zweite Schicht (99) überlagert und mit dieser verbunden ist.

7. Der medizinische Verband nach Anspruch 6, wobei der Abschnitt (10a) des Körpers (10) den ersten Einschnitt (24) und den zweiten Einschnitt (26) enthält.

8. Der medizinische Verband nach Anspruch 6 oder 7, wobei der Abschnitt (10a) des Körpers (10) eine Rahmenstruktur bildet, die eine Grenze um einen Abschnitt der ersten Schicht (98) bildet und ein transparentes oder durchscheinendes Fenster in dem Verband bildet, das eine Beobachtung von mindestens einem Abschnitt des zentralen Bereichs (18) ermöglicht.

9. Der medizinische Verband nach einem der Ansprüche 6 bis 8, wobei die erste Schicht (98) einen ersten Bereich definiert, wobei die zweite Schicht (99) einen zweiten Bereich definiert, wobei der erste Bereich mindestens 50 % des zweiten Bereichs beträgt.

10. Der medizinische Verband nach Anspruch 9, wobei der erste Bereich mindestens 90 % des zweiten Bereichs beträgt.

11. Der medizinische Verband nach einem der Ansprüche 6 bis 8, wobei die erste Schicht (98) einen ersten Bereich definiert, wobei die zweite Schicht (99) einen zweiten Bereich definiert, wobei der zweite Bereich mindestens 50 % des ersten Bereichs beträgt.

12. Der medizinische Verband nach einem der Ansprüche 6 bis 8, wobei die erste Schicht (98) einen ersten Bereich definiert, wobei die zweite Schicht (99) einen zweiten Bereich definiert, wobei der zweite Bereich mindestens 90 % des ersten Bereichs beträgt.

13. Der medizinische Verband nach einem der vorstehenden Ansprüche, wobei der erste Einschnitt (24) einen ersten Schlitzabschnitt aufweist.

14. Der medizinische Verband nach einem der vorstehenden Ansprüche, wobei der zweite Einschnitt (26) einen zweiten Schlitzabschnitt aufweist.

15. Der medizinische Verband nach einem der vorstehenden Ansprüche, wobei der Körper (10) ferner einen fünften Einschnitt (44) aufweist, der sich von dem Umfang (16) in die oder angrenzend an die erste seitliche Erstreckung (30) erstreckt.

## Revendications

1. Pansement médical (100, 102, 104, 106, 108, 109, 200, 600, 601, 602, 603, 604, 605), comprenant :
un corps (10) comprenant :
une première surface principale (12) ;
une seconde surface principale (14) opposée à la première surface principale ;
un périmètre (16) ;
une région centrale (18) ;
un premier rabat (20) défini par une première extrémité distale (22) formée par une première partie (16a) du périmètre, une première indentation (24) ayant une première extrémité (25) à proximité de la région centrale, une seconde indentation (26) ayant une seconde extrémité (27) à proximité de la région centrale, et une première région charnière (28) s'étendant entre la première extrémité et la seconde extrémité ;
dans lequel le premier rabat a une longueur maximale (y) et une largeur maximale (x) ;
dans lequel un rapport (r) de la largeur maximale à la longueur maximale est inférieur à 4 à 1 ;
une première extension latérale (30) s'étendant dans une première direction (S) à l'écart du premier rabat ; et
une seconde extension latérale (32) s'étendant dans une seconde direction (T) à l'écart du premier rabat ; la première direction étant sensiblement opposée à la seconde direction ;
un adhésif (40) disposé sur au moins une partie de la seconde surface principale ;
la partie de la seconde surface principale sur laquelle l'adhésif est disposé comportant une partie de chaque premier rabat, première extension latérale et seconde extension latérale.

2. Pansement médical selon la revendication 1 :
dans lequel le premier rabat (20) définit un premier axe de rabat (29) s'étendant du périmètre (16) à la première région charnière (28) ;
dans lequel, à proximité de la première extrémité (25), la première indentation (24) comprend une partie (124) qui s'étend dans une direction à l'écart du premier axe de rabat.

3. Pansement médical selon la revendication 1 ou la revendication 2 :
dans lequel le premier rabat (20) définit un premier axe de rabat (29) s'étendant du périmètre (16) à la première région charnière (28) ;
dans lequel, à proximité de la seconde extrémité (27), la seconde indentation (26) comprend une partie (126) qui s'étend dans une direction à l'écart du premier axe de rabat.

4. Pansement médical selon l'une quelconque des revendications précédentes, dans lequel le corps (10) comprend un matériau en feuille choisi dans le groupe constitué d'un film polymère transparent, d'un film polymère translucide, d'un film polymère opaque, d'un tissu tissé, d'un tissu non tissé, d'une mousse, et d'une combinaison de deux ou plusieurs des matériaux en feuille susmentionnés.

5. Pansement médical selon la revendication 4, dans lequel le matériau en feuille est autoportant.

6. Pansement médical selon la revendication 4, dans lequel au moins une partie (10a) du corps (10) comprend une première couche (98) superposée et liée à une seconde couche (99).

7. Pansement médical selon la revendication 6, dans lequel la partie (10a) du corps (10) comporte la première indentation (24) et la seconde indentation (26).

8. Pansement médical selon la revendication 6 ou la revendication 7, dans lequel la partie (10a) du corps (10) forme une structure de cadre qui forme une bordure autour d'une partie de la première couche (98) et fournit une fenêtre transparente ou translucide dans le pansement qui permet une observation d'au moins une partie de la région centrale (18).

9. Pansement médical selon l'une quelconque des revendications 6 à 8, dans lequel la première couche (98) définit une première zone, dans lequel la seconde couche (99) définit une seconde zone, la première zone étant au moins 50 % de la seconde zone.

10. Pansement médical selon la revendication 9, dans lequel la première zone est au moins 90 % de la seconde zone.

11. Pansement médical selon l'une quelconque des revendications 6 à 8, dans lequel la première couche (98) définit une première zone, dans lequel la seconde couche (99) définit une seconde zone, la seconde zone étant au moins 50 % de la première zone.

12. Pansement médical selon l'une quelconque des revendications 6 à 8, dans lequel la première couche (98) définit une première zone, dans lequel la seconde couche (99) définit une seconde zone, la seconde zone étant au moins 90 % de la première zone.

13. Pansement médical selon l'une quelconque des revendications précédentes, dans lequel la première indentation (24) comprend une première partie fendue.

14. Pansement médical selon l'une quelconque des revendications précédentes, dans lequel la seconde indentation (26) comprend une seconde partie fendue.

15. Pansement médical selon l'une quelconque des revendications précédentes, dans lequel le corps (10) comprend en outre une cinquième indentation (44) s'étendant du périmètre (16) dans ou adjacente à la première extension latérale (30).
